(19) Europäisches Patentamt
European Patent Office
Office européen des brevets

(11) EP 1 157 029 B1

(12) **EUROPÄISCHE PATENTSCHRIFT**

(45) Veröffentlichungstag und Bekanntmachung des Hinweises auf die Patenterteilung:
**28.12.2005 Patentblatt 2005/52**

(21) Anmeldenummer: **99903574.4**

(22) Anmeldetag: **23.02.1999**

(51) Int Cl.[7]: **C07K 5/083**, C07K 5/087, C07K 5/103, C07K 5/107, G01N 33/68

(86) Internationale Anmeldenummer:
**PCT/CH1999/000082**

(87) Internationale Veröffentlichungsnummer:
**WO 2000/050446 (31.08.2000 Gazette 2000/35)**

(54) **OLIGOPEPTIDDERIVATE ZUR ELEKTROCHEMISCHEN MESSUNG DER AKTIVITÄT VON PROTEASEN**

OLIGOPEPTIDE DERIVATIVES FOR THE ELECTROCHEMICAL MEASUREMENT OF PROTEASE ACTIVITY

DERIVES D'OLIGOPEPTIDES POUR LA MESURE ELECTROCHIMIQUE DE L'ACTIVITE DE PROTEASES

(84) Benannte Vertragsstaaten:
**AT CH DE DK ES FR GB IT LI NL SE**

(43) Veröffentlichungstag der Anmeldung:
**28.11.2001 Patentblatt 2001/48**

(73) Patentinhaber: **Pentapharm AG**
**4002 Basel (CH)**

(72) Erfinder:
- **LUDIN, Christian**
  **CH-4153 Reinach (CH)**
- **WIKSTROEM, Peter**
  **CH-5073 Gipf-Oberfrick (CH)**
- **SVENDSEN, Lars, G.**
  **CH-4153 Reinach (CH)**
- **SCHULZE, Andreas**
  **CH-4147 Aesch (CH)**

(74) Vertreter: **Braun, André jr.**
**Braunpat Braun Eder AG**
**Patent - Marken - Rechtsanwälte**
**Reussstrasse 22**
**Postfach**
**4015 Basel (CH)**

(56) Entgegenhaltungen:
**EP-A- 0 018 002**
**EP-A- 0 034 122**
**EP-A- 0 152 872**
**EP-A- 0 182 373**
**DE-A- 3 428 543**
**US-A- 4 505 852**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 157 029 B1

## Beschreibung

Die vorliegende Erfindung betrifft Oligopeptidderivate der Formel (I)

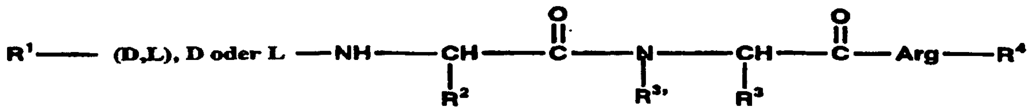

worin

$R^1$ (a) ein Wasserstoffatom, eine gegebenenfalls in ω-Stellung eine Aminogruppe tragende $C_{2-8}$-Alkanoylgruppe, eine Phenyl--$C_{2-4}$-alkanoylgruppe, deren Phenylrest gegebenenfalls in p-Stellung mit einer Aminogruppe substituiert ist; oder

(b) eine gegebenenfalls in 4-Stellung mit einem Aminomethylrest substituierte Cyclohexylcarbonylgruppe, eine gegebenenfalls in o-oder p-Stellung mit Methyl, Amino, oder Halogen substituierte Benzoylgruppe, eine $C_{1-8}$-Alkoxycarbonylgruppe, eine gegenbenfalls in p-Stellung mit Methoxy, Methyl oder Chlor substituierte Benzyloxycarbonylgruppe; oder

(c) einen Rest der Formel $-SO_2-R^5$, wobei $R^5$ ein $C_{1-6}$-Alkylrest, ein gegebenenfalls substituierter Aryl- oder Heteroarylrest oder ein Rest eines bicyclischen Terpenderivats sein kann; oder

(d) eine Gruppe der Formel $-CO-CH(R^6)-NH-R^7$, wobei $R^6$ Wasserstoff, einen $C_{1-6}$-Alkylrest, einen 1- oder 2-Hydroxyethylrest, einen Methylmercaptoethylrest, einen Aminobutylrest, einen Guanidinopropylrest, einen Carboxy-$C_{1-}$-$_4$-alkylrest, einen Carboxamido-$C_{1-4}$-alkylrest, einen Phenyl-$C_{1-4}$-alkylrest, dessen Phenylrest gegebenenfalls mit OH, Halogen, $C_{1-4}$-Alkyl oder Methoxy substituiert ist, einen Cyclohexyl- oder Cyclohexylmethylrest, dessen Ring gegebenfalls mit OH, Halogen, $C_{1-4}$-Alkyl oder Methoxy substituiert ist, oder einen stickstoffhaltigen Heteroaryl-$C_{1-4}$-alkylrest mit 3 bis 8 Kohlenstoffatomen im heterocyclischen System bezeichnet, wobei die Gruppe $-CO-CH(R^6)-NH-R^7$ racemisch oder D-bzw. L-konfiguriert sein kann und $R^7$ eine Gruppe des Typs (a), (b), oder (c) sein kann; oder

(e) eine Gruppe der Formel

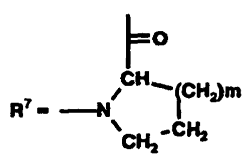

wobei $R^7$ obige Bedeutung besitzt, m 1 oder 2 sein kann und eine der Methylengruppen durch Hydroxyl, Carboxyl, $C_{1-4}$-Alkyl oder Aryl-$C_{1-4}$-alkyl substituiert sein kann;

$R^2$ Wasserstoff, $C_{1-6}$-Alkyl, $C_{1-2}$-Hydroxyalkyl, $C_{1-4}$-Alkoxy-$C_{1-6}$-alkyl, Benzyloxy-$C_{1-2}$-alkyl, einen (ω-Carboxy-$C_{1-3}$-alkylrest, einen ω-C-$_{1-4}$-Alkoxycarbonyl-$C_{1-3}$ -alkylrest, einen ω-Benzyloxycarbonyl-$C_{1-3}$-alkylrest oder einen Cyclohexyl-Cyclohexylmethyl-, 4-Hydroxycyclohexylmethyl-, Phenyl-, Benzyl-, 4-Hydroxybenzyl- oder Imidazolyl-4- methylrest;

$R^3$ (a) Wasserstoff oder $C_{1-4}$-Alkyl und $R^{3'}$ Wasserstoff; oder
(b) zusammen mit $R^{3'}$ eine Tri- oder Tetramethylengruppe, wobei eine der Methylengruppen durch Hydroxyl, Carboxyl, $C_1$-$_4$-Alkyl oder Aryl-$C_{1-4}$-alkyl substituiert sein kann; und

$R^4$ wobei $R^8$ Hydroxy und $R^9$ Wasserstoff, Halogen, Amino, Nitro, $C_{1-4}$-Alkyl, $C_{1-4}$-Alkoxy oder $C_{1-4}$-Alkanoyl sein

können; oder

(a) einen Anilinrest der Formel

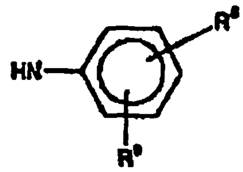

(b) einen Chinolinrest der Formel

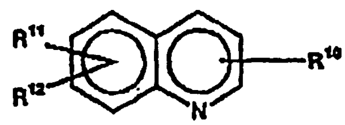

wobei eines von $R^{10}$, $R^{11}$ und $R^{12}$ eine -NH-Gruppe darstellt, über welche der Chinolinrest mit dem Arg-Rest verknüpft ist, ein zweites Hydroxy oder Amino sein kann und das dritte Wasserstoff, Hydroxy oder Amino sein kann;

bedeuten, und deren Salze.

Diese Oligopeptidderivate und deren Salze werden von Enzymen der Enzymklasse der Peptidhydrolasen (E. C. 3.4.), insbesondere Proteinasen (B.C. 3.4.21 - 99) und deren Inhibitoren, des Blutgerinnungssystems, des fibrinolytischen Systems und des Komplements, insbesondere des Thrombins, gespalten. Sie dienen damit als Substrate zur quantitativen und qualitativen Bestimmung der o.g. Enzyme, insbesondere Thrombin, in komplexen Probenflüssigkeiten, insbesondere Kapillarblut.

EP-A-0 034 122 beschreibt zur Bestimmung von Enzymen geeignete Tripeptidderivate mit L-Arginin als C-terminale Aminosäure, welche in Form von Amiden mit gewissen aromatischen oder heterocyclischen Aminen vorliegt, wobei u.a. folgende Aminogruppen spezifisch genannt werden: p-Nitrophenylamino; 1-Carboxy-2-nitrophenyl-(5)-amino; 1-Sulfo-2-nitrophenyl-(5)-amino; Chinolyl-(5)-amino; und 8-Nitrochinolyl-(5)-amino.

US-A-4 505 852 beschreibt zur Bestimmung von Enzymen und Enzyminhibitoren geeignete Amide von 7-Amino-4-trifluormethylchinolon mit Aminosäuren oder mit zwei oder mehrere Aminosäuren enthaltenden Peptiden.

EP-A-0 182 373 beschreibt einen Gerinnungstest auf Teststreifen unter Verwendung von in bestimmter Weise substituierten Aniliden von N-terminal geschütztem Arginin oder Lysin oder von N-terminal geschützten Di-, Tri- oder Tetrapeptiden mit Arg oder Lys als C-terminale Aminosäure. Diese Anilide sind in o-oder p- Stellung durch $NR_1R_2$ substituiert, wobei $R_1$ und $R_2$ u.a. Wasserstoff bedeuten können; zusätzlich können diese Anilide gegebenenfalls noch durch eine Carboxylester- oder Carboxylamidogruppe, ein Halogenatom, eine Nitrogruppe oder eine $C_{1-3}$-Alkylgruppe substituiert sein.

DE-A-34 28 543 beschreibt zur Bestimmung von Proteasen des Blutgerinnungssystems geeignete Substrate, welche mit den Aniliden gemäss EP-A 0 182 373 weitgehend übereinstimmen.

SP-A-0 152 872 beschreibt das Arginyl-3-carboxy-4-hydroxyanilid als Ausgangsprodukt für Substrate zur Messung von Enzymaktivitäten, wie D-Phe-Pro-Arg-3-carboxy 4-hydroxyanilid.

Gegenstand der vorliegenden Erfindung sind die Oligopeptidderivate der Formel (I) und deren Salze, die Herstellung dieser Oligopeptidderivate und Salze sowie ein Verfahren zur quantitativen Bestimmung einer Protease oder Antiprotease, insbesondere des Blutgerinnungssystems, des fibrinolytischen Systems oder des Komplements, im besonderen Thrombin, welches mittels der Oligopeptidderivate oder deren Salzen durchgeführt wird.

Der in dieser Beschreibung verwendete Ausdruck "Alkyl", für sich allein genommen oder in Kombinationen, wie "Hydroxyalkyl" oder "Benzyloxyalkyl", bezeichnet geradkettige oder verzweigte gesättigte Kohlenwasserstoffreste, wie Methyl, Ethyl, n-Propyl, Isopropyl, n-Butyl, Isobutyl, sek.-Butyl, tert.-Butyl u.dgl. Der Ausdruck "Alkoxy" bezeichnet einen über eine Sauerstoffbrücke verknüpften Alkylrest im Sinne der vorstehenden Definition von "Alkyl". Der Ausdruck

"Alkanoyl" bezeichnet den Acylrest einer Alkylcarbonsäure, welche geradkettig oder verzweigt sein kann, wie Acetyl, Propionyl u. dgl. Der Ausdruck "Aryl" bezeichnet den Rest eines aromatischen Kohlenwasserstoffs und umfasst Reste, wie Phenyl, Naphthyl u. dgl. Der Ausdruck "Heteroaryl" bezeichnet den Rest eines aromatischen heterocyclischen Systems, wie Imidazolyl, Indolyl, Chinolinyl, Isochinolinyl u. dgl.

Wenn in Formel (I) $R^1$ einen Rest der Formel $-SO_2R^5$ bedeutet, dann kann $R^5$ beispielsweise Methyl, Isopropyl, Phenyl, tert.-Butylphenyl, 4-Methylphenyl, 2,4,6-Trimethylphenyl, 2,4,6-Triisopropylphenyl, 4-Methoxy-2,3,6-trimethylphenyl, Anthrachinoyl, 1- oder 2-Naphtyl, Chinolyl oder Isochinolyl oder ein von Campher abgeleiteter Rest sein. Bedeuten in Formel (I) $R^1$ eine Gruppe der Formel $-CO-CH(R^6)-NH-R^7$ und $R^6$ Heteroarylalkyl, dann kann $R^6$ beispielsweise Imidazolylmethyl oder Indolylmethyl sein.

Wenn im Molekül die N-terminale Aminosäure eine Schutzgruppe trägt, dann ist diese Schutzgruppe zweckmässigerweise tert.-Butoxycarbonyl "(Boc"), p-Toluolsulfonyl ("Tos"), tert.-Butylphenylsulfonyl ("t-Bups"), Methylsulfonyl ("Mes"), Naphthylsulfonyl ("Naps"), Benzoyl ("Bzo"), Benzyloxycarbonyl ("Z"), Isopropylsulfonyl oder Camphersulfonyl.

Wenn $R^4$ einen Anilinrest bedeutet, dann sind zweckmässigerweise $R^8$ Hydroxy in o- oder p-Position und $R^9$ Halogen in m-Position oder $R^8$ Hydroxy in o-, m- oder p-Position und $R^9$ Nitro oder $C_{1-4}$-Alkanoyl in m- oder p-Position. Vorzugsweise ist der Rest $R^4$ von 2-Amino-4-nitrophenol, 4-Amino-2-nitrophenol, 4-Amino-3-nitrophenol, 2-Amino-5-nitrophenol, 2,4- Diaminophenol, 4-Amino-m-cresol, 2-Amino-4-chlorophenol, 4-Amino-2-chlorophenol, 4-Amino-3-chlorophenol, 4-Fluoro-2-aminophenol, 2-Fluoro-4-aminophenol, 5-Fluoro-2-aminophenol, 5-Amino-8-hydroxychinolin oder 2-Amino-8-hydroxychinolin abgeleitet.

Die oligopeptidderivate der Formel (I) enthalten als C-terminale Aminosäure L-Arginin ("Arg"). Weitere Aminosäuren, welche im Molekül vorhanden sein können, sind beispielsweise 2-Aminobuttersäure, Alanin, 3-Cyclohexylalanin, 2-Cyclohexylglycin, Phenylalanin, Pipecolinsäure, Prolin und Valin, wobei diese Aminosäuren in L-, D- oder DL-Form vorliegen können, sowie Glycin; vorzugsweise handelt es sich dabei um L-Alanin ("Ala"), L-2-Aminobuttersäure ("Abu"), D-3-cyclohexylalanin ("D-Cha") D-2-Cyclohexylglycin ("D-Chg"), Glycin ("Gly") und L-Prolin("Pro").

Repräsentative Beispiele von Oligopeptidderivaten der Formel (I) sind:

H-(D)-Chg-Gly-Arg-3-chloro-4-hydroxyanilid;
Boc-(D)-Chg-Gly-Arg-3-chloro-4-hydroxyanilid;
Tos-(D)-Chg-Gly-Arg-3-chloro-4-hydroxyanilid;
t-Bups-(D)-Chg-Gly-Arg-3-chloro-4-hydroxyanilid;
Mes-(D)-Chg-Gly-Arg-3'-chloro-4-hydroxyanilid;
Naps-2-(D)-Chg-Gly-Arg-3-chloro-4-hydroxyanilid;
Z-(D)-Chg-Gly-Arg-3-chloro-4-hydroxyanilid;
H-(D)-Cha-Gly-Arg-3-chloro-4-hydroxyanilid;
Boc-(D)-Cha-Gly-Arg-3-chloro-4-hydroxyanilid;
H-Gly-Pro-Arg-3-chloro-4-hydroxyanilid:
Boc-Gly-Pro-Arg-3-chloro-4-hydroxyanilid;
Tos-Gly-Pro-Arg-3-chloro-4-hydroxyanilid;
t-Bups-Gly-Pro-Arg-3-chloro-4-hydroxyanilid;
Mes-Gly-Pro-Arg-3-chloro-4-hydroxyanilid;
Isopropylsulfonyl-Gly-Pro-Arg-3-chloro-4-hydroxyanilid;
Naps-2-Gly-Pro-Arg-3-chloro-4-hydroxyanilid;
(-)-Camphersulfonyl-Gly-Pro-Arg-3-chloro-4-hydroxyanilid;
H-(D)-Cha-Pro-Arg-3-chloro-4-hydoxyanilid;
Boc-(D)-Cha-Pro-Arg-3-chloro-4-hydroxyanilid;
H-(D)-Cha-Ala-Arg-3-chloro-4-hydroxyanilid;
Boc-(D)-Cha-Ala-Arg-3-chloro-4-hydroxyanilid;
Boc-(D)-Cha-Abu-Arg-3-chloro-4-hydroxyanilid;
Z-Gly-Pro-Arg-2-chloro-4-hydroxyanilid;
Z-Gly-Pro-Arg-5-chloro-2-hydroxyanilid;
Z-Gly-Pro-Arg-8-hydroxychinolin-5-ylamid;
Boc-(D)-Chg-Gly-Pro-Arg-3-chloro-4-hydroxyanilid:
H-(D)-Chg-Gly-Pro-Arg-3-chloro-9-hydroxyanilid; und
t-Bups-(D)-Chg-Gly-Pro-Arg-3-chloro-9-hydroxyanilid.

Im Blutstrom gelangen zahlreiche Proteinasen an ihren Wirkort im Organismus. Dies unterstreicht die Bedeutung der Kenntnis der Enzymaktivität für das Verständnis verschiedenster Krankheitsbilder. Störungen der Balance zwischen Enzym und Inhibitor sind für den Ausbruch einer Krankheit verantwortlich. Deshalb ist es von unschätzbarem

Wert, die vorliegende Enzymaktivität bestimmen zu können.

Blut oder Plasma sind jedoch als Analysemedien wegen ihrer Eigenschaften (Viskosität, Absorption von sichtbarem Licht) nicht einfach zu handhaben. Meist werden Enzymtests mittels photometrischer Methoden durchgeführt. Hier stösst man im besonderen an Grenzen bei Blut, Plasma oder Serum.

In der Regel verlangen photometrische Detektionsmethoden klare Probenlösungen, da die chromogenen Eigenschaften der verwendeten synthetischen Substrate und deren Chromophore sonst nicht zur Wirkung kommen. Dies bedingt oft ein Vorschalten von Reinigungsschritten, um zu einer messbaren Lösung zu kommen (vgl. DE 3616496 A1 und DE 19549117 A1).

Messungen der Enzyme des Blutgerinnungssystems werden z.B. gewünscht und durchgeführt bei Patienten mit Herzklappenersatz oder nach Herzinfarkten. Derartige Patienten werden einer Therapie mit oralen Antikoagulantien unterzogen. Es sollen damit weitere thromboembolische Ereignisse verhindert werden. Zu diesem Zweck werden die Patienten in regelmässigen Abständen mit Medikamenten aus der Klasse der Coumarin-Derivate behandelt. Diese Therapie bedarf allerdings einer Überwachung zur optimalen Dosierung. Der Parameter der Wahl, um ein optimale Dosierung ableiten zu können, ist die Bestimmung der Thromboplastinzeit (PT) nach Quick.

Mittels der Oligopeptidderivate gemäss vorliegender Erfindung wird es möglich, die Thromboplastinzeit mittels eines Substrates, welches ein elektrochemisches Signal erzeugt, zu bestimmen. Weiterhin ist es möglich, ein hinreichend gutes Signal aus Kapillarblut zu erzeugen.

In der europäischen Patentschrift EP 0018002 B1 wird ein elektrochemisches Verfahren zur Bestimmung von Proteasen der Blutgerinnungsysteme, insbesondere von Anti-Thrombin, in Citratplasma mittels amperogener Substrate beschrieben. Leider sind bei diesem eleganten Verfahren zur direkten Bestimmung von Thrombin bzw. Trypsin Messungenauigkeiten möglich, da die Zugabe eines Lösungsvermittlers (DMSO) zum Reaktionsmedium notwendig ist, damit das amperogene Substrat im Reaktionsmedium gelöst bleibt (J. M. Nigretto et. al., Thrombosis Research 20, 299-306, 1980; Thrombosis Research 22, 303-308). Die Zugabe eines Kosolvens kann für eine praktische Anwendung, z.B. Screening im komplexen System Blutplasma oder Blut, z.B. durch Gerinnungsauslösung, Kompromittierung eines Enzyms in der Kaskade, lokale Ausfällungseffekte des Substrats o.dgl., zu einer Ungenauigkeit in der Messung von Thrombin führen.

Die Oligopeptidderivate gemäss vorliegender Erfindung ermöglichen nun ein direktes amperogenes Bestimmungsverfahren von Thrombin, das an die Praxis besser angepasst ist als die bisher bekannten Bestimmungsverfahren. Gemäss diesem Verfahren misst man die Zunahme einer wassserlöslichen amperogenen Verbindung, welche bei der Hydrolyse des Substrats mit dem Enzym entsteht. Dabei wird ein Peptidrest, dessen C-terminaler Peptidrest L-Arginin ist, und ein elektroaktiver Rest, welcher über eine Amidbindung mit dem L-Arginin verbunden ist, durch das Enzym gespalten. Der elektroaktive Rest wird dann elektrochemisch oxidiert oder reduziert, die Änderung des Stromes wird gemessen, und dieser ist proportional zur Konzentration des amperogenen Restes, der während der enzymatischen Hydrolyse gebildet wird.

Das erfindungsgemässe Verfahren ermöglicht die quantitative Bestimmung einer Protease oder Antiprotease, insbesondere des Blutgerinnungssystems, des fibrinolytischen Systems oder des Komplements, im besonderen Thrombin; es ist dadurch gekennzeichnet, dass man in einem wässrigen oder organischen Medium das Enzym mit einem Oligopeptidderivat der Formel (I) oder einem Salz davon zusammenbringt und das durch das zu bestimmende Enzym abgespaltene elektroaktive Amin der Formel $H\text{-}R^4$, worin $R^4$ obige Bedeutung besitzt, welches elektrochemisch oxidiert oder reduziert werden kann, mittels Amperometrie bestimmt.

Zweckmässigerweise wird die Amperometrie mittels eines Geräts mit einem Potentiostat und einer Messzelle mit zwei oder drei Elektroden, einer Messelektrode aus Stahl oder einem Edelmetall, wie Platin oder Gold, einer Bezugselektrode, und/oder einer Hilfselektrode ausgeführt, wobei das Oligopeptidderivat der Formel (I) oder dessen Salz und eventuelle wasserlösliche Zusätze, wie $Ca^{++}$-Salze, Phopholipide oder Thromboplastinreagentien, auf den Elektroden aufgebracht ist bzw. sind.

Die Veränderung der Konzentration des elektroaktiven Amins der Formel $R^4\text{-}H$, worin $R^4$ obige Bedeutung besitzt, kann in der Messzelle durch den gemessenen Oxidations- oder Reduktionsstrom bestimmt werden.

In einer bevorzugten Ausführungsform des erfindungsgemässen Bestimmungsverfahrens ist eine direkte Haftung dieser wasserlöslichen Substrate und von eventuellen wasserlöslichen Zusätzen, wie z. B. $Ca^{++}$, Phospholipide, Innovin® (Firma DADE-Behring), auf einer leitenden Oberfläche aus Stahl oder Edelmetall möglich, wobei die erforderlichen Elektroden durch einen isolierten Steg getrennt sind. Nach Zugabe von Medien, welche Thrombin enthalten oder welches zuvor generiert worden ist, z.B. im Blut oder Blutplasma mit Aktivatoren, z.B. $Ca^{++}$, Phospholipide, Thromboplastinreagentien o.dgl., ist das Substrat vollständig gelöst, so dass durch die Freisetzung der amperogenen Aminoverbindung eine direkte Bestimmung des Stroms, proportional zu der Konzentration der elektroaktiven Aminoverbindung, und damit die Bestimmung der Enzymkonzentration im Medium möglich ist.

Überraschend ist, dass als Phospholipide im Gegensatz zu EP 0 565 665 vorzugsweise ein Gemisch aus 0-35% L-$\alpha$-Phosphatidylcholin (PC) und 65-100% L-$\alpha$-Phosphatidyl-L-Serin (PS) verwendet wird.

Ein besonders bevorzugtes Mischverhältnis der Phospholipide PC : PS beträgt 65 bis 75% PS und 25 bis

35% PC.

Das erfindungsgemässe Bestimmungsverfahren kann zur Bestimmung von Thrombin in Vollblut verwendet werden: Vollblut wird direkt, ohne Abtrennung der Blutzellen und ohne Abtrennung von anderen Blutbestandteilen, auf eine mit einem Oligopeptidderivat der Formel(I)oder mit einem Salz davon und mit eventuellen wasserlöslichen Zusätzen behaftete Elektrode aufgebracht.

Das Substrat des erfindungsgemässen Bestimmungsverfahrens, nämlich ein Oligopeptidderivat der Formel (I), besteht aus einem Oligopeptidrest, an welchen der Rest eines wasserlöslichen aromatischen oder heteroaromatischen Amins gebunden ist. Diese Reste von aromatischen oder heteroaromatischen Aminen tragen zusätzliche funktionelle Gruppen, wie z.B. Hydroxygruppen oder Halogenatome, welche vorteilhaft zu einem tieferen elektrochemischen Potential beitragen und die Löslichkeit des Substrats in wässrigem Medium entscheidend bestimmen.

Die Oligopeptidderivate der Formel (I) und deren Salze können erfindungsgemäss dadurch hergestellt werden, dass man nach in der Peptidchemie üblichen Methoden (allgemeine Vorschriften von M. Bodanszky "The Practice of Peptide Synthesis" Springer Verlag, $2^{nd}$ Edition 1994) ein Amin der Formel $H\text{-}R^4$, worin $R^4$ obige Bedeutung besitzt, mit der Carboxlgruppe von Arginin, dessen Aminogruppe geschützt ist oder bereits den entsprechend geschützten Rest des Peptidteils des gewünschten Produkts oder eines Teils desselben trägt und dessen Argininingruppe geschützt ist, verknüpft und notwendigenfalls den Peptidteil des gewünschten Produkts vollständig aufbaut, worauf man erwünschtenfalls die verbleibende(n) Schutzgruppe(n) abspaltet und erwünschtenfalls eine freie Aminogruppe acyliert und/oder erwünschtenfalls ein erhaltenes Oligopeptidderivat der obigen Formel I in ein Säureadditionssalz und/ oder ein erhaltenes Säureadditionssalz eines solchen Oligopeptidderivats in ein freies Oligopeptidderivat oder in ein anderes Salz überführt.

Beispielsweise kann man so vorgehen, dass man die elektroaktive Aminogruppe an die Carboxylgruppe des C-terminalen Arginins anknüpft, wobei dessen Aminogruppe durch eine Schutzgruppe, z.B. eine Benzyloxycarbonyl- oder tert.-Butyloxycarbonylgruppe, und die Guanidinogruppe des Arginins durch Protonisierung, z.B. mit HCl oder p-Toluolsulfonsäure, geschützt werden. Die C-terminale elektroaktive Aminogruppe dient während des stufenweisen Aufbaus der Peptidkette ebenfalls als Schutzgruppe. Die anderen Schutzgruppen können je nach Bedarf selektiv abgespalten werden, um die weiteren Aminosäurereste anzuknüpfen, bis die gewünschte Kettenlänge vollständig aufgebaut ist. Zum Schluss können die verbleibenden Schutzgruppen vollständig abgespalten werden, ohne dass die elektroaktive Aminogruppe in Mitleidenschaft gezogen wird.

Oligopeptidderivate mit freier N-terminaler Aminogruppe können acyliert werden, beispielsweise mit t-Butylphenylsulfonylchlorid, Tosylchlorid, Acetylchlorid, Butylchlorid, Octanoylchlorid, Benzoesäurechlorid, p-Methylbenzoesäurechlorid, 2-Chlorbenzoesäurechlorid, Methylsulfonylchlorid, n-Butylsulfonylchlorid, t-Butylsulfonylchlorid, Isopropylsulfonylchlorid, Phenylsulfonylchlorid, 1- oder 2-Naphthylsulfonylchlorid, (+)-oder (-)-Camphersulfonylchlorid oder mit Malonsäurechlorid.

Bevorzugte Salze von Oligopeptidderivaten der Formel (I) sind solche, worin die stark basische Guanidinogruppe des Arginins durch Protonisierung mit HCl, HBr, $H_2SO_4$ oder $H_3PO_4$ oder mit Ameisen-, Essig-, Propion-, Phthal-, Zitronen-, Oxal-, Wein-, Benzoe-, Milch-, Trichloressig- oder Trifluoressigsäure stabilisiert ist, insbesondere mit HCl, Essigsäure oder Trifluoressigsäure.

Beim erfindungsgemässen Bestimmungsverfahren spaltet das zu bestimmende Enzym die Bindung zwischen der carboxyterminalen Aminosäure Arginin und dem wasserlöslichen aromatischen oder heterocyclischen Amin. Das Messverfahren besteht darin, dass die Menge des freigesetzen Amins ("elektroaktive Spezies") elektrochemisch bestimmt wird. Dieses elektrochemische Bestimmungsverfahren eignet sich zur Bestimmung von Proteasen der Blutgerinnungssysteme, insbesondere von Thrombin.

Das Substrat dieses Bestimmungsverfahrens besteht z.B. aus Tosyl-glycyl-prolyl-arginyl-3-chloro-4-hydroxyanilid-MonoAcetat-Salz, wobei folgende enzymatische Reaktion stattfindet:

Substrat: Tos-Gly-Pro-Arg-3-chloro-4-hydroxyanilid x AcOH

\+ Enzym ↓ Thrombin

Produkt: Tos-Gly-Pro-Arg-OH + 4-Amino-2-chlorophenol

Der besondere Vorteil des erfindungsgemässen Bestimmungsverfahrens besteht darin, dass die Substrate ohne Zusatz von Lösungsvermittlern, wie z.B. DMSO, homogen in einem beliebigen wässrigen Reaktionsmedium gelöst sind. Mit diesem elektrochemischen Bestimmungsverfahren lässt sich daher mit hoher Messgenauigkeit die Enzymkonzentration im Reaktionsmedium bestimmen.
Eine zweckmässige Ausführungsart des erfindungsgemässen Bestimmungsverfahrens wird nachstehend anhand der beiliegenden Zeichnungen näher erläutert.
Es zeigen:

| | |
|---|---|
| Fig. 1 | ein Voltammogramm von 4-Amino-2-chlorophenol; |
| Fig. 2 | ein Voltammogramm von 5-Amino-8-hydroxychinolin; |
| Fig. 3 | die lineare Abhängigkeit der Stromstärke von der Konzentration von 4-Amino-2-chlorphenol, 5-Fluoro-2-aminophenol und 5-Amino-8-hydroxychinolin; |
| Fig. 4 | ein Cyclovoltammigramm und die Hydrolyse von Tos-Gly-Pro-Arg-3-chloro-4-hydroxyanilid x AcOH; |
| Fig. 5 - 7 | die linearen Spaltraten dreier verschiedener Substrate; |
| Fig. 8 | die linearen Spaltraten des Substrats Tos-Gly-Pro-Arg-3-chloro-4-hydroxyanilid x AcOH mit verschiedenen Thrombinkonzentrationen; |
| Fig. 9 | schematische Darstellung einer Messanordnung; |
| Fig. 10 | eine weitere Messkurve entsprechend Fig. 8 mit 10 µl Thrombin bei 300 mV; |
| Fig. 11 | schematische Darstellung einer weiteren Messanordnung und |
| Fig. 12 | weitere Messkurven entsprechend Fig. 10 mit 10 µl Vollblut bei 500 mV. |

**I. Benutzte Geräte:**

1) Benutzt wird ein Potentiostat Tacusell PGP201, Software VM1, der einen konstanten Potentialunterschied zwischen der Messelektrode und der Bezugselektrode aufrecht erhält. Als Messzelle dient ein Becherglas mit einem Inhalt von 25 ml oder 1 ml, das die 3 Elektroden aufnehmen kann.
2) Als Messelektrode dient ein Platindraht mit der Oberfläche 34 $mm^2$ oder 3 $mm^2$.
3) Als Bezugselektrode wird eine Kalomelelektrode (SCE) verwendet, auf welcher die Spannungswerte gemessen werden.
4) Die Hilfselektrode besteht aus Platindraht und dient dazu, den Durchgang des Stroms zu gewährleisten.
5) Die Messlösung wird zwecks Homogenisierung mittels eines Magnetrührers gerührt.

**II. Aufzeichnung der zyklischen Voltammogramme:**

1) In einem 25 ml Becherglas wird zunächst der Untergrundstrom des Puffers gemessen mit einem Cyclus von -30 bis 600 mv mit 500 mV/min. Wenn der Grundstrom eine hinreichende Genauigkeit ergibt, kann ein Messzyklus stattfinden. Der Grundstrom wird vom Messwert während der Hydrolyse eines Substrates oder der Aufnahme eines Profils einer elektroaktiven Verbindung abgezogen.
2) Auswertung der Ergebnisse: Die im Linearitätsbereich aufgenommenen Stromwerte sind nach Abzug des Grundstromes bei dem betreffenden Potential proportional zur Konzentration der elektroaktiven Spezies während der Hydrolyse. Die freigesetzte elektroaktive Spezies, welche durch das Substrat abgespalten wird, ist ebenfalls proportional zur eingesetzten Enzymkonzentration.
3) Mit einer Platinelektrode werden die cyclischen Voltammogramme bei Konzentrationen von 0.25 mmol, 0.5 mmol und 1.0 mmol aufgenommen. Als Puffer wird HEPES, 50 mmol bei pH 7.4 verwendet. Figuren 1 und 2 zeigen typische cyclische reversible Kurven von 4-Amino-2-chlorophenol und 5-Amino-8-hydroxychinolin: Aufgenommen wurde von -30 bis 600 mV bei 500 mV/min. Vorzugsweise zeigen diese amperogenen Substanzen eine lineare Abhängigkeit der Stromstärke von der Konzentration bei tiefem Potential im Bereich von 300 mV, bei einer hohen Stromstärke. Das System befindet sich hier im Gleichgewicht. Eine höhere Spannung > 400 mV ist wegen Interferenzen mit möglichen Bestandteilen im Blut, z.B. Paracetamol, nicht erwünscht.

Figur 3 zeigt die lineare Abhängigkeit der Stromstärke von der Konzentration von 4-Amino-2-chlorophenol, 5-Fluoro-2-aminophenol und 5-Amino-8-hydroxychinolin bei Konzentrationen von 0.25 mmol, 0.5 mmol und 1.0 mmol in HEPES-Puffer bei 300 mV mittels Doppelbestimmung.
Figur 4 (gepunktete Linie) zeigt das potentiodynamische Cyclovoltammigramm in einer Messzelle von 1 ml

Volumen und Elektrodenoberfläche von 3 $mm^2$ von Tos-Gly-Pro-Arg-3-chloro-4-hydroxyanilid x AcOH bei -30 bis 600 mV /500 mV/m bei c = 0.5 mM im HEPES-Puffer, pH = 7.4, 0.1 M KCl: Vorteilhaft bei 400 mV zeigt das Substrat im Gleichgewichtsbereich von der elektroaktiven Verbindung 4-Amino-2-chlorophenol keine Elektroaktivität, auch nicht vom peptidischen Teil des Substrats Tos-Gly-Pro-Arg-OH. Im Vergleich zum Cyclovoltammigramm im 25 ml Becherglas wird die Gleichgewichtslage erst bei 400 mV erreicht, was auf das Messergebniss keinen Einfluss hat.

Figur 4 (durchgezogene Linie) zeigt die Hydrolyse von Tos-Gly-Pro-Arg-3-chloro-4-hydroxyanilid x AcOH mit 1 NIH-U Thrombin und c = 0.5 mM. Das potentiodynamische Cyclovoltammigramm bei 400 mV zeigt das Substrat, die Hydrolyseprodukte Tos-Gly-Pro-Arg-OH und die elektroaktive Spezies 4-Amino-2-chlorophenol. Interferenzen der Komponenten Tos-Gly-Pro-Arg-OH oder Tos-Gly-Pro-Arg-3-chloro-4-hydroxyanilid x AcOH mit der zu bestimmenden elektroaktiven Spezies 4-Amino-2-chlorophenol sind nicht ersichtlich, so dass eine einwandfreie Bestimmung der elektroaktiven Spezies 4-Amino-2-chlorophenol und somit der Enzymkonzentration möglich ist.

**III. Messung der Enzymaktivität von Thrombin in Lösung:**

Experimentelle Bedingungen:
Thrombin 5000 NIH-U, Diagnotec Liestal 100-500 Thrombinkonzentration: 0.5, 1.0, 2 NIH-U/ ml
Temperatur: 25 °C
Puffer: HEPES-Puffer, 50 mM, pH 7.4, KCl, 0.1 M
Vorbereitung:
Es wird z.B. eine 0.5 mM Substratlösung Tos-Gly-Pro-Arg-3-chloro-4-hydroxyanilid x AcOH im HEPES-Puffer hergestellt, sowie Thrombinstammlösung von 100 NIH-U/1 mL in dest. Wasser.

In einer 1 ml Messzelle, versehen mit 3 Elektroden des Potentiostats Tacusell PGP201, Software VM1, werden 1 ml Substrat-Lösung (c = 0.5 mmol) vorgelegt. Man lässt ein Cycle potentiodynamisch von -30 bis 500 mV zur Aufnahme des Nullwertes laufen (10 min). Zur Messung legt man 10µl Thrombinlösung (1.0 NIH-U/ml Endkonzentration) in der Messzelle vor, gibt 990 µl Substratlösung zu, mischt 30 sec und nimmt den Strom potentiostatisch über eine Zeit von 10 min auf. Die Messungen werden als Doppelbestimmung durchgeführt.

Figuren 5, 6 und 7 (µA $cm^2$/min, T = 0 - 5 min, bei 400 mV) zeigen die linearen Spaltraten anhand von 3 Substraten mit unterschiedlichen elektroaktiven Gruppen bei der Thrombinkonzentation von 1 NIH-U und Substratkonzentration c = 0.5 mmol:

1) Tos-Gly-Pro-Arg-3-chloro-4-hydroxyanilid x AcOH,
2) H-(D)-Chg-Gly-Arg-3-chloro-4-hydroxyanilid x 2 TFA,
3) Z-Gly-Pro-Arg-2-chloro-4-hydroxyanilid x HCl.

Figur 8 (µA$cm^2$/min, bei 400 mV) zeigt die linearen Spaltraten des Substrats Tos-Gly-Pro-Arg-3-chloro-4-hydroxyanilid x AcOH (c = 0.5 mM) mit Thrombinkonzentrationen von 0.5, 1 und 2 NIH-U (es wurde eine Doppelbestimmung durchgeführt).

Alle Beispiele zeigen eine einwandfreie lineare Messung von Thrombin bei unterschiedlichen Thrombinkonzentrationen im wässrigen Medium mit unterschiedlichen elektroaktiven Gruppen.

Tabelle 1

| Substrat | Freigesetzte elektroaktive Spezies | Standardisierung |
|---|---|---|
| 1): Tos-Gly-Pro-Arg-3-chloro-4-hydroxyanilid x AcOH | 4-Amino-2-chloro-phenol | Die Hydrolyse des Substrats (c = 0.5 mmol) mit 1 NIH-U Thrombin ergibt einen Strom von 2 µA/$cm^2$ Elektrodenoberfläche in einer Minute |
| 2): H-(D)-Chg-Gly-Arg-3-chloro-4-hydroxyanilid x 2 TFA | 4-Amino-2-chloro-phenol | Die Hydrolyse des Substrats (c = 0.5 mmol ) mit 1 NIH-U Thrombin ergibt einen Strom von 3 µA/$cm^2$ Elektrodenoberfläche in einer Minute |

Tabelle 1 (fortgesetzt)

| Substrat | Freigesetzte elektroaktive Spezies | Standardisierung |
|---|---|---|
| 3): Z-Gly-Pro-Arg-2-chloro-4-hydroxyanilid x HCl | 4-Amino-3-chlorophenol | Die Hydrolyse des Substrats (c = 0.5 mmol ) mit 1 NIH-U Thrombin ergibt einen Strom von 0.5 μA/cm$^2$ Elektrodenoberfläche in einer Minute |

**IV. Messung der Enzymaktivität von Thrombin auf einer Metalloberfläche:**

Figur 9 zeigt die Messanordnung.
In einer 1 ml Messzelle wird eine 0.5 mM Substratlösung in Puffer (HEPES, 50 mmol, pH 7.4, KCl 0.1 M) gegeben. In diese tauchen 2 Elektroden mit einer Metalloberfläche (Messelektrode Pt, kombinierte Bezugs-/Hilfselektrode Ag/AgCl). Man verbindet die Elektroden mit einem Potentiostat, z.B. Tacusell PGP201 mit Software VM1 (Hersteller: Radiometer). Zur Aufnahme des Nullwertes wird ein Zyklus potentiodynamisch von -30 bis 500 mV während 5 min aufgenommen. Zur Messung gibt man eine Lösung von Thrombin mit z.B. 0.5 oder 1 oder 2 NIH-U zu. Man misst nach 10 sec über einen Zeitraum von 5 min den Strom, bei konstantem Potential von 300 mV. Die Messungen werden als Doppelbestimmungen durchgeführt.

Figur 10 zeigt die lineare Spaltraten des Substrats Tos-Gly-Pro-Arg-3-chloro-4-hydroxyanilid x AcOH (μA cm$^2$/min, T = 0 - 5 min, bei 300 mV) mit 10 μl Thrombin mit 0.5, 1 und 2 NIH-U. Eine Messung von Thrombin ist daher mit der elektroaktiven Gruppe ohne Interferenzen mit dem Substrat oder dem abgespaltenen Peptidrest einwandfrei möglich. Der Kurvenverlauf der Substrathydrolyse stimmt mit den Ergebnissen der Substrathydrolyse mit 0.5, 1 und 2 NIH-U Thrombin in der 1 mL Messzelle überein.

Eine solche Messung kann auch auf einem Sensorstreifen wie folgt durchgeführt werden:

Auf einem Sensorstreifen werden 10 μl einer 0.5 mM Substratlösung, gelöst in dem oben erwähnten Puffer, aufgetragen und getrocknet. Die Elektroden des Sensors werden verbunden mit einem Potentiostat Tacussel PGP201, Software VM 1. Zur Messung bringt man 10 μl einer Lösung von Thrombin mit z.B. 0.5 oder 1 oder 2 NIH-U auf die mit Substrat behaftete Metalloberfläche auf, wobei sich das Substrat homogen löst. Man verfolgt den Strom über einen Zeitraum von 5 min bei konstantem Potential von 300 mV.

Dieses Vorgehen ist detailliert beschrieben in der am gleichen Tag hinterlegten EP-Patentanmeldung der Asulab SA, Marin/Schweiz, mit dem Titel "Système électrochimique pour la determination d'un temps de coagulationdu sang".

**V. Messung der Enzymaktivität von Thrombin im Vollblut auf einer Metalloberfläche:**

Messanordung:

Auf einer Metalloberfläche 1 mit der Fläche 3.5 mm$^2$ (Figur 11) verbunden mit zwei Elektroden 2 eines Potentiostats 3 Tacusell PGP201, Software VM1 werden 10 μl einer 0.5 mmol Substrat-thromboplastinlösung aufgetragen und 2 h bei 30 °C getrocknet. Die Substrat-Thromboplastinlösung kann z.B. auf folgende Weise hergestellt werden: Käufliches rekombinantes tissue-Factor(Fa. American Diagnostica) wurde mit BSA-Lösung (1 mg/ml) versetzt und mit HEPES, 50 mmol, pH 7.4, KCl 0.1 M verdünnt. Dazu gab man eine Mischung aus L-α-Phosphatidylcholin und L-α-Phosphatidyl-L-Serin in Na-Deoxycholat gelöst. Zu dieser Lösung gab man das Substrat Tos-Gly-Pro-Arg-3-chloro-4-hydroxyanilid x AcOH, so dass die Substrat-Thromboplastinlösung 0.5 mmol Konzentration an amperogenen Substrat enthält und 25% L-α-Phosphatidylcholin (PC) und 75% L-α-Phosphatidyl-L-Serin (PS). Das Verhältnis von Phospholipiden zu Tissue-Factor beträgt 1:10000. Zur Messung bringt man 10 μl Vollblut auf die mit Substrat-Thromboplastinlösung behaftete Metalloberfläche 1 auf, wobei sich das Substrat homogen löst. Man misst den Strom nach 10 sec über einen Zeitraum von 3 min. bei konstantem Potential von 500 mV. Die Messungen werden als Dreifachbestimmung durchgeführt.

Figur 12 zeigt die Spaltraten des Substrats Tos-Gly-Pro-Arg-3-chloro-4-hydroxyanilid x AcOH (μA cm$^2$/min, T = 0 - 3 min., bei 500 mV) mit 10 μl Vollblut als Dreifachbestimmung (Kurven 1 - 3 sowie Puffer, Gerade 4).

In den nachfolgenden Ausführungsbeispielen, welche die Erfindung illustrieren, ihren Umfang aber in keiner Weise einschränken sollen, wird die Herstellung von erfindungsgemässen Oligopeptidderivaten der Formel (I) und von Salzen solcher Oligopeptid-derivate beschrieben. Die Analyse der gemäss den Beispielen erhaltenen Eluate und Produkte wurde mit Proton-NMR, HPLC-Elektrospray-MS oder Elementaranalyse ausgeführt.

Benutzte Abkürzungen:

Ala: L-Alanin
Arg: L-Arginin
Abu: L-2-Aminobuttersäure
D-Cha: D-3-Cyclohexylalanin
D-Chg: D-2-Cyclohexylglycin
Gly: Glycin
Pro: L-Prolin
AcOH: Essigsäure
Boc: tert.-Butoxycarbonyl
t-Bups: t-Butyl-phenylsulfonyl
DCH: N,N-Dicyclohexylharnstoff
DCC: Dicyclohexylcarbodiimid
DMF: N,N-Dimethylformamid
NHS: N-Hydroxysuccinimid
HCl: Chlorwasserstoff
Mes: Methylsulfonyl
Naps-2-: 2-Naphthylsulfonyl
NMM: N-Methylmorpholin
TBTU: O-(Benzotriazol-1-yl)-N,N,N',N',-tetramethyluronium-tetrafluoroborat
TFA: Trifluoressigsäure
Tos: p-Toluolsulfonyl
Z: Benzyloxycarbonyl
RT: Raumtemperatur
BSA: Bovin Albumin Serum

Beispiel 1:

Boc-(D)-Chg-Gly-Pro-Arg-3-chloro-4-hydroxyanilid x TFA

**1a:** Boc-Arg-3-chloro-4-hydroxyanilid:

32.1 g (98.0 mmol) käufliches Boc-(L)-Arg-OH x HCl x $H_2O$ wurden in 150 ml DMF gelöst, auf 0°C abgekühlt und mit 12.84 g (107.0 mmol) Isobutylchloroformat versetzt. Nach 1 min wurden 10.8 g (107.0 mmol) NMM zugegeben. Anschliessend wurden 14.0 g (98.0 mmol) 4-Amino-2-chlorophenol zugefügt und die Lösung bei 0°C 4 h gerührt. Nach Einengen und Extraktion (1-Butanol/Wasser) und säulenchromatogaphischer Reinigung (Sephadex® LH 20) erhielt man 34.0 g (76.0%) Produkt.

| Elementaranalyse: $C_{19}H_{27}N_5O_6F_3Cl$ | | | |
|---|---|---|---|
| Ber | C 44.41 | H 5.30 | N 13.63 |
| Gef | C 44.0 | H 5.43 | N 13.82 |

**1b**: H-Arg-3-chloro-4-hydroxyanilid x 2 TFA

34.0 g (75.0 mmol) la wurden mit 20% Trifluoressigsäure/Dichlormethan bei RT 3 h gerührt. Nach Einengen und nach säulenchromatographischer Reinigung (Sephadex® LH 20) erhielt man 31.1 g (76.0%) Produkt.

**1c**: Boc-(D)-Chg-Gly-OH

8.1 g (31.5 mmol) Boc-(D)-Chg-OH wurden in 100 ml 1,2-Dimethoxyethan gelöst, worauf bei 0°C NHS (3.7 g, 32.0 mmol) und DCC (7.0 g, 34.0 mmol) zugegeben wurden. Nach 6 h bei RT wurde DCH abfiltriert, und zum Filtrat gab man eine Lösung aus 2.3 g (31.0 mmol) Glycin und 2.6 g (31.0 mmol) $NaHCO_3$ in 350 ml Wasser. Nach 24 h Rühren bei RT engte man ein und extrahierte mit 5% Zitronensäure/Ethylacetat. Nach Einengen der organischen Phase erhielt man 8.2 g (85.0%) Produkt.

**1d**: Boc-(D)-Chg-Gly-Pro-Arg-3-chloro-4-hydroxyanilid x TFA

4.0 g, (12.7 mmol) **1c** wurden in 50 ml DMF gelöst, worauf 4.2 g (13.0 mmol) TBTU und 3.9 g (39.0 mmol) NMM zugegeben wurden. Zur klaren Lösung gab man 6.9 g (12.7 mmol) **1b**. Nach 5h bei RT engte man ein und extrahierte mit n-Butanol/Wasser. Nach Einengen der organischen Phase und säulenchromatographischer Reinigung (Sephadex® LH 20) erhielt man 7.0 g (68.0%) Produkt.

Beispiel 2:

H-(D)-Chg-Gly-Pro-Arg-3-chloro-4-hydroxyanilid x 2 TFA

6.8 g (8.4 mmol) 1d wurden in 20% Trifluoressigsäure/Dichlormethan bei RT 3 h gerührt. Nach Einengen und säulenchromatographischer Reinigung (Sephadex® LH 20) erhielt man 5.0 g (72.0%) Produkt.

Beispiel 3:

t-Bups-(D)-Chg-Gly-Pro-Arg-3-chloro-4-hydroxyanilid x TFA

1.2 g (1.46 mmol) 2 wurden in 20 ml DMF gelöst, worauf 0.339 g (1.46 mol) t-Butylbenzolsulfonylchlorid und 0.332 g (2.92 mmol) NMM zugegeben wurden. Nach 20 h Rühren bei RT wurde eingeengt, und nach säulenchromatographischer Reinigung (Sephadex® LH 20) erhielt man 1.1 g (83.0%) Produkt.

Anstelle von t-Butylphenylsulfonylchlorid kann man als Acylierungsmittel auch z.B. die entsprechenden Mengen Tosylchlorid, Acetylchlorid, Butylchlorid, Octanoylchlorid, Benzoesäurechlorid, p-Methylbenzoesäurechlorid, 2-Chlorbenzoesäurechlorid, Methylsulfonylchlorid, n-Butylsulfonylchlorid, t-Butylsulfonylchlorid, Isopropylsulfonylchlorid, Phenylsulfonylchlorid, 1- oder 2-Naphthylsulfonylchlorid, (+)-oder (-)-Camphersulfonylchlorid oder Malonsäurechlorid verwenden, wobei man entsprechend acylierte Derivate von 2 erhält.

Beispiel 4:

t-Bups -(D)-Chg-Gly-Pro-Arg-3-chloro-4-hydroxyanilid x AcOH

0.47 g (0.52 mmol) 3 wurden im Lösungsmittelgemisch Methanol/Wasser 6:4 gelöst und auf eine Ionenaustauschersäule (Acetatform) gegeben, worauf eluiert wurde. Das Eluat wurde eingeengt, und nach säulenchromatographischer Reinigung (Sephadex® LH 20) erhielt man 0.388 g (88.0%) Produkt.

Das Substrat kann auch mit einer Mineralsäure, z.B. HCl, HBr, $H_2SO_4$ oder $H_3PO_4$, oder mit einer organischen Säure, z.B. Ameisensäure, Oxalsäure oder Weinsäure, protonisiert werden, wobei man entsprechende Salze von 3 erhält.

Beispiel 5:

Tos-Gly-Pro-Arg-3-chloro-4-hydroxyanilid x AcOH

1.8 g (5.5 mmol) Tos-Gly-Pro-OH wurden gemäss Beispiel 1d mit 1.94 g (6.0 mmol) TBTU und 1.8 g (18 mmol) NMM aktiviert und mit 2.38 g (5.5 mmol) **1b** umgesetzt. Das erhaltene Tos-Gly-Pro-Arg-3-chloro-4-hydroxyanilid x TFA (3.6 g, 4.90 mmol, 90%) wurde gemäss Beispiel 4 über Ionenaustauscher in das Acetat-Salz überführt, Ausbeute 2.9 g (90.0%).

| Elementaranalyse: $C_{28}$ $H_{38}$ $N_7$ $O_8$ $S_1$ $F_3$ Cl | | | |
|---|---|---|---|
| Ber | C 50.33 | H 5.73 | N 14.67 |
| Gef | C 48.76 | H 5.85 | N 14.26 |

Beispiel 6:

Z-Gly-Pro-Arg-8-hydroxychinolin-5-ylamid x HCl

1.0 g (2.0 mmol) Z-Gly-Pro-Arg-OH x HCl wurden analog Beispiel 1a mit 0.2 g (2.1 mmol) Isobutylchloroformat und 0.466 g (4.0 mmol) NMM aktiviert und mit 0.466 g (2.0 mmol) 5-Amino-8-hydroxychinolin x 2 HCl zu 1.12 g (72.0%)

Produkt umgesetzt.

Beispiel 7:

Z-Gly-Pro-Arg-2-chloro-4-hydroxyanilid x HCl

1.0 g (2.0 mmol) Z-Gly-Pro-Arg-OH x HCl wurden analog Beispiel 1a mit 0.2 g (2.1 mmol) Isobutylchloroformat und 0.466 g (4.0 mmol) NMM aktiviert und mit 0.36 g (2.0 mmol) 4-Amino-3-chlorophenol x HCl zu 0.875 g (70.0%) Produkt umgesetzt.

## Patentansprüche

1. Oligopeptidderivate der Formel (I)

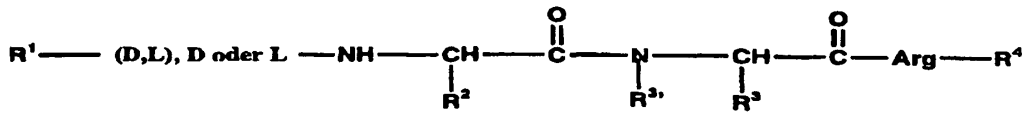

worin

$R^1$ (a) ein Wasserstoffatom, eine gegebenenfalls in ω-Stellung eine Aminogruppe tragende $C_{2-9}$-Alkanoylgruppe, eine Phenyl-$C_2$-$_4$-alkanoylgruppe, deren Phenylrest gegebenfalls in p-Stellung mit einer Aminogruppe substituiert ist; oder

(b) eine gegebenenfalls in 4-Stellung mit einem Aminomethylrest substituierte Cyclohexylcarbonylgruppe, eine gegebenenfalls in o-oder p-Stellung mit Methyl, Amino, oder Halogen substituierte Benzoylgruppe, eine $C_{1-8}$-Alkoxycarbonylgruppe, eine gegenbenfalls in p-Stellung mit Methoxy, Methyl oder Chlor substituierte Benzyloxycarbonylgruppe; oder

(c) einen Rest der Formel -$SO_2$-$R^5$, wobei $R^5$ ein $C_{1-6}$-Alkylrest, ein gegebenenfalls substituierter Aryl- oder Heteroarylrest oder ein Rest eines bicyclischen Terpenderivats sein kann; oder

(d) eine Gruppe der Formel -CO-CH($R^6$)-NH-$R^7$, wobei $R^6$ Wasserstoff, einen $C_{1-6}$-Alkylrest, einen 1- oder 2-Hydroxyethylrest, einen Methylmercaptoethylrest, einen Aminobutylrest, einen Guanidinopropylrest, einen Carboxy-$C_{1-4}$-alkylrest, einen Carboxamido-$C_{1-4}$-alkylrest, einen Phenyl-$C_{1-4}$-alkylrest, dessen Phenylrest gegebenenfalls mit OH, Halogen. $C_{1-4}$-Alkyl oder Methoxy substituiert ist, einen Cyclohexyloder Cyclohexylmethylrest, dessen Ring gegebenfalls mit OH, Halogen, $C_{1-4}$-Alkyl oder Methoxy substituiert ist, oder einen stickstoffhaltigen Heteroaryl-$C_{1-4}$-alkylrest mit 3 bis 8 Kohlenstoffatomen im heterocyclischen System bezeichnet, wobei die Gruppe -CO-CH($R^6$)-NH-$R^7$ racemisch oder D-bzw. L-konfiguriert sein kann und $R^7$ eine Gruppe des Typs (a), (b), oder (c) sein kann; oder

e) eine Gruppe der Formel

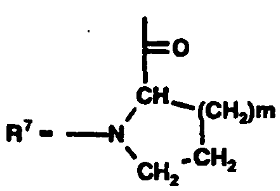

wobei $R^7$ obige Bedeutung besitzt, m 1 oder 2 sein kann und eine der Methylengruppen durch Hydroxyl, Carboxyl, $C_{1-4}$-Alkyl oder Aryl-$C_{1-4}$-alkyl substituiert sein kann;

$R^2$ Wasserstoff, $C_{1-6}$-Alkyl, $C_{1-2}$-Hydroxyalkyl, $C_{1-4}$-Alkoxy-$C_{1-6}$-alkyl, Benzyloxy-$C_{1-2}$-alkyl, einen ω-Carboxy-$C_{1-3}$-alkylrest, einen ω-C-$_{1-4}$-Alkoxycarbonyl-$C_{1-3}$ -alkylrest, einen ω-Benzyloxycarbonyl-$C_{1-3}$-alkylrest oder einen Cyclohexyl-Cyclohexylmethyl-, 4-Hydroxycyclohexylmethyl-, Phenyl-, Benzyl-, 4-Hydroxybenzyl- oder Imidazolyl-4- methylrest;

$R^3$ (a) Wasserstoff oder $C_{1-4}$-Alkyl und $R^{3'}$ Wasserstoff; oder
(b) zusammen mit $R^{3'}$ eine Tri- oder Tetramethylengruppe,
wobei eine der Methylengruppen durch Hydroxyl, Carboxyl, $C_{1-4}$-Alkyl oder Aryl-$C_{1-4}$-alkyl substituiert sein kann; und

$R^4$ (a) einen Anilinrest der Formel

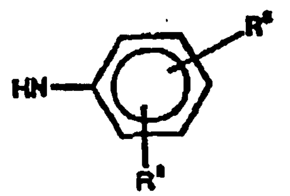

wobei $R^8$ Hydroxy und $R^9$ Wasserstoff, Halogen, Amino, Nitro, $C_{1-4}$-Alkyl, $C_{1-4}$-Alkoxy oder $C_{1-4}$-Alkanoyl sein können; oder

(b) einen Chinolinrest der Formel

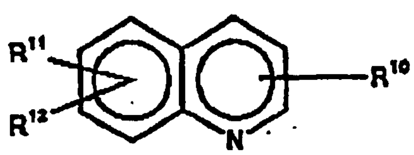

wobei eines von $R^{10}$, $R^{11}$ und $R^{12}$ eine -NH-Gruppe darstellt, über welche der Chinolinrest mit dem Arg-Rest verknüpft ist, ein zweites Hydroxy oder Amino sein kann und das dritte Wasserstoff, Hydroxy oder Amino sein kann;

bedeuten, und deren Salze.

**2.** Verbindungen gemäss Anspruch 1, worin $R^5$ Methyl, Isopropyl, Phenyl, tert.-Butylphenyl, 4-Methylphenyl, 2,4,6-Trimethylphenyl, 2,4,6-Triisopropylphenyl, 4-Methoxy-2,3,6-trimethylphenyl, Anthrachinoyl, 1- oder 2-Naphtyl, Chinolyl oder Isochinolyl oder ein von Campher abgeleiteter Rest ist bzw. $R^4$ in der Bedeutungsmöglichkeit Heteroarylalkyl Imidazolylmethyl oder Indolylmethyl ist und/oder worin $R^8$ Hydroxy in o- oder p-Position und $R^9$ Halogen in m-Position oder $R^8$ Hydroxy in o-, m- oder p-Position und $R^9$ Nitro oder $C_{1-4}$-Alkanoyl in m- oder P-Position sind.

**3.** Verbindungen gemäss Anspruch 1 oder 2, worin die N-terminale Aminosäure eine Schutzgruppe trägt, und zwar tert.-Butoxycarbonyl "(Boc"), p-Toluolsulfonyl ("Tos"), tert.-Butylphenylsulfonyl ("t-Bups"), Methylsulfonyl ("Mes"), Naphthylaulfonyl ("Naps"), Benzoyl ("Bzo"), Benzyloxycarbonyl ("Z"), Isopropylsulfonyl oder Camphersulfonyl.

**4.** Verbindungen gemäss einem der Ansprüche 1 bis 3, worin das Molekül, ausser der C-terminalen Aminosäure L-Arginin, 2-Aminobuttersäure, Alanin, 3-Cyclohexylalanin, 2-Cyclohexylglycin, Phenylalanin, Pipecolinsäure, Prolin oder Valin, wobei diese Aminosäuren in L-, D- oder DL-Form vorliegen können, oder Glycin enthält.

**5.** Verbindungen gemäss Anspruch 4, worin das Molekül, ausser der C-terminalen Aminosäure L-Arginin, L-Alanin ("Ala"), L-2-Aminobuttersäure ("Abu"), D-3-Cyclohexylalanin ("D-Cha"), D-2-Cyclohexylglycin ("D-Chg"), Glycin ("Gly") oder L-Prolin ("Pro") enthält.

**6.** Verbindungen gemäss einem der Ansprüche 1 bis 5, worin $R^4$ von 2-Amino-4-nitrophenol, 4-Amino-2-nitrophenol, 4-Amino-3-nitrophenol, 2-Amino-5-nitrophenol, 2,4-Diaminophenol, 4-Amino-m-cresol, 2-Amino-4-chlorophenol, 4-Amino-2-chlorophenol, 4-Amino-3-chlorophenol, 4-Fluoro-2-aminophenol, 2-Fluoro-4-aminophenol, 5-Fluoro-2-aminophenol, 5-Amino-8-hydroxychinolin oder 2-Amino-8-hydroxychinolin abgeleitet ist.

**7.** H-(D)-Chg-Gly-Arg-3-chloro-4-hydroxyanilid;

Boc-(D)-Chg-Gly-Arg-3-chloro-4-hydroxyanilid;
Tos-(D)-Chg-Gly-Arg-3-chloro-4-hydroxyanilid;
t-Bups-(D)-Chg-Gly-Arg-3-chloro-4-hydroxyanilid;
Mes-(D)-Chg-Gly-Arg-3-chloro-9-hydroxyanilid;
Naps-2-(D)-Chg-Gly-Arg-3-chloro-4-hydroxyanilid;
Z-(D)-Chg-Gly-Arg-3-chloro-4-hydroxyanilid;
H-(D)-Cha-Gly-Arg-3-chloro-9-hydroxyanilid;
Boc-(D)-Cha-Gly-Arg-3-chloro-4-hydroxyanilid;
H-Gly-Pro-Arg-3-chloro-4-hydroxyanilid:
Boc-Gly-Pro-Arg-3-chloro-4-hydroxyanilid;
Tos-Gly-Pro-Arg-3-chloro-4-hydroxyanilid;
t-Bups-Gly-Pro-Arg-3-chloro-4-hydroxyanilid;
Mes-Gly-Pro-Arg-3-chloro-4-hydroxyanilid;
Isopropylsulfonyl-Gly-Pro-Arg-3-chloro-4-hydroxyanilid;
Naps-2-Gly-Pro-Arg-3-chloro-4-hydroxyanilid;
(-)-Camphersulfonyl-Gly-Pro-Arg-3-chloro-4-hydroxyanilid;
H-(D)-Cha-Pro-Arg-3-chloro-4-hydoxyanilid;
Boc-(D)-Cha-Pro-Arg-3-chloro-4-hydroxyanilid;
H-(D)-Cha-Ala-Arg-3-chloro-4-hydroxyanilid;
Boc-(D)-Cha-Ala-Arg-3-chloro-4-hydroxyanilid;
Boc-(D)-Cha-Abu-Arg-3-chloro-4-hydroxyanilid;
Z-Gly-Pro-Arg-2-chloro-4-hydroxyanilid;
Z-Gly-Pro-Arg-5-chloro-2-hydroxyanilid;
Z-Gly-Pro-Arg-8-hydroxychinolin-5-ylamid;
Boc-(D)-Chg-Gly-Pro-Arg-3-chloro-4-hydroxyanilid;
H-(D)-Chg-Gly-Pro-Arg-3-chloro-4-hydroxyanilid; und
t-Bups-(D)-Chg-Gly-Pro-Arg-3-chloro-4-hydroxyanilid.

**8.** Salze von Verbindungen gemäss einem der Ansprüche 1 bis 7 mit HCl, HBr, $H_2SO_4$ oder $H_3PO_4$ oder mit Ameisen-, Essig-, Propion-, Phthal-, Zitronen-, Oxal-, Wein-, Benzoe-, Milch-, Trichloressig- oder Trifluoressigsäure.

**9.** Salze gemäss Anspruch 8 mit HCl, Essigsäure oder Trifluoressigsäure.

**10.** Verfahren zur Herstellung von Verbindungen und Salzen gemäss einem der Ansprüche 1 bis 9, **dadurch gekennzeichnet, dass** man nach in der Peptidchemie üblichen Methoden ein Amin der Formel H-$R^4$, worin $R^4$ die in Anspruch 1 angegebene Bedeutung besitzt, mit der Carboxlgruppe von Arginin, dessen Aminogruppe geschützt ist oder bereits den entsprechend geschützten Rest des Peptidteils des gewünschten Produkts oder eines Teils desselben trägt und dessen Argininguppe geschützt ist, verknüpft und notwendigenfalls den Peptidteil des gewünschten Produkts vollständig aufbaut, worauf man erwünschtenfalls die verbleibende(n) Schutzgruppe(n) abspaltet und erwünschtenfalls eine freie Aminogruppe acyliert und/oder erwünschtenfalls ein erhaltenes Oligopeptidderivat der in Anspruch 1 definierten Formel I in ein Säureadditionssalz und /oder ein erhaltenes Säureadditionssalz eines solchen Oligopeptidderivats in ein freies Oligopeptidderivat oder in ein anderes Salz überführt.

**11.** Verfahren zur quantitativen Bestimmung einer Protease oder Antiprotease, **dadurch gekennzeichnet, dass** man in einem wässrigen oder organischen Medium das Enzym mit einem Oligopeptidderivat oder einem Salz davon gemäss einem der Ansprüche 1 bis 9 zusammenbringt und das durch das zu bestimmende Enzym abgespaltene elektroaktive Amin der Formel H-$R^4$, worin $R^4$ die in Anspruch 1 angegebene Bedeutung besitzt, welches elektrochemisch oxidiert oder reduziert werden kann, mittels Amperometrie bestimmt.

**12.** Verfahren gemäss Anspruch 11, **dadurch gekennzeichnet, dass** man als Enzym eine Protease oder Antiprotease des Blutgerinnungssystems, des fibrinolytischen Systems oder des Komplements, im besonderen Thrombin, mit dem Oligopeptidderivat oder einem Salz davon zusammenbringt.

**13.** Verfahren gemäss Anspruch 11 oder 12, **dadurch gekennzeichnet, dass** die Amperometrie mittels eines Gerätes mit einem Potentiostat und einer Messzelle mit zwei oder drei Elektroden, einer Messelektrode aus Stahl oder einem Edelmetall, wie Platin oder Gold, einer Bezugselektrode, und/oder einer Hilfselektrode ausgeführt wird, wobei das Oligopeptidderivat oder dessen Salz gemäss einem der Ansprüche 1 bis 9 und eventuelle wasserlösliche Zusätze, wie $Ca^{++}$-Salze, Phospholipide oder Thromboplastinreagentien, auf den Elektroden aufgebracht ist bzw. sind.

**14.** Verfahren gemäss Anspruch 13, **dadurch gekennzeichnet, dass** als Phospholipide ein Gemisch von 0-35% L-$\alpha$-Phosphatidylcholin (PC) und 65-100 % L-$\alpha$-Phosphatidyl-L-Serin (PS) verwendet wird.

**15.** Verfahren gemäss Anspruch 13, **dadurch gekennzeichnet, dass** das Gemisch 25-35 % PC und 65-75 % PS enthält.

**16.** Verfahren gemäss einem der Ansprüche 13 bis 15, **dadurch gekennzeichnet, dass** die Verändererung der Konzentration des elektroaktiven Amins in der Messzelle durch den gemessenen Oxidations- oder Reduktionsstrom bestimmt wird.

**17.** Verfahren gemäss einem der Ansprüche 11 bis 16 zur Bestimmung von Thrombin in Vollblut, **dadurch gekennzeichnet, dass** das Vollblut direkt, ohne Abtrennung der Blutzellen und ohne Abtrennung von anderen Blutbestandteilen, auf die mit dem Oligopeptidderivat oder dessen Salz gemäss einem der Ansprüche 1 bis 9 und mit eventuellen wasserlöslichen Zusätzen behaftete Elektrode aufgebracht wird.

## Claims

**1.** Oligopeptide derivatives of the formula (I)

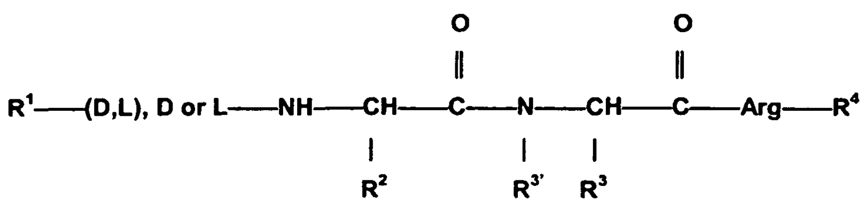

in which

$R^1$ is

(a) a hydrogen atom, a $C_{2-8}$-alkanoyl group optionally having an amino group in the W position, a phenyl-$C_{2-4}$-alkanoyl group whose phenyl radical is optionally substituted in the p position by an amino group; or

(b) a cyclohexylcarbonyl group which is optionally substituted in the 4 position by an aminomethyl radical, a benzoyl group which is optionally substituted in the o or p position by methyl, amino or halogen, a $C_{1-8}$-alkoxycarbonyl group, a benzyloxycarbonyl group which is optionally substituted in the p position by methoxy, methyl or chlorine; or

(c) a radical of the formula $-SO_2-R^5$ wherein $R^5$ can be a $C_{1-6}$-alkyl radical, an optionally substituted aryl or heteroaryl radical or a radical of a bicyclic terpene derivative; or

(d) a group of the formula -CO-CH($R^6$)-NH-$R^7$, wherein $R^6$ is hydrogen, a $C_{1-6}$-alkyl radical, a 1- or 2-hydroxyethyl radical, a methylmercaptoethyl radical, an aminobutyl radical, a guanidinopropyl radical, a carboxy-$C_{1-4}$-alkyl radical, a carboxamido-$C_{1-4}$-alkyl radical, a phenyl-$C_{1-4}$-alkyl radical whose phenyl radical is optionally substituted by OH, halogen, $C_{1-4}$-alkyl or methoxy, or a cyclohexyl or cyclohexylmethyl radical whose ring is optionally substituted by OH, halogen, $C_{1-4}$-alkyl or methoxy, or a nitrogen-containing heteroaryl-$C_{1-4}$-alkyl radical with 3 to 8 carbon atoms in the heterocyclic system, wherein the group -CO-CH ($R^6$) -NH-$R^7$may be racemic or have the D or L configuration, and $R^7$ can be a group of type (a), (b) or (c) ; or

(e) a group of the formula

$R^7$ = 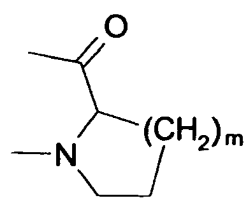

wherein $R^7$ has the denotation mentioned above, m can be 1 or 2, and one of the methylene groups can be substituted by OH, carboxyl, $C_{1-4}$-alkyl or aryl-$C_{1-4}$-alkyl;

$R^2$ is hydrogen, $C_{1-6}$-alkyl, $C_{1-2}$-hydroxyalkyl, $C_{1-4}$-alkoxy-$C_{1-6}$-alkyl, benzyloxy-$C_{1-2}$-alkyl, an ω-carboxy-$C_{1-3}$-alkyl radical, an ω-$C_{1-4}$-alkoxycarbonyl-$C_{1-3}$-alkyl radical, an ω-benzyloxycarbonyl-$C_{1-3}$-alkyl radical or a cyclohexyl, cyclohexylmethyl, 4-hydroxycyclohexylmethyl, phenyl, benzyl, 4-hydroxybenzyl or imidazolyl-4-methyl radical;

$R^3$ is

(a) hydrogen or $C_{1-4}$-alkyl and $R^{3'}$ is hydrogen; or

(b) together with $R^{3'}$ a tri- or tetramethylene group, where one of the methylene groups can be substituted by OH, carboxyl, $C_{1-4}$-alkyl or aryl-$C_{1-4}$-alkyl; and

$R^4$ is (a) an aniline residue of the formula

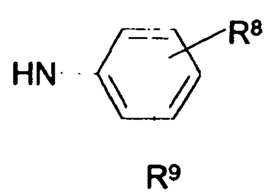

where $R^8$ can be OH and $R^9$ can be hydrogen, halogen, amino, nitro, $C_{1-4}$-alkyl, $C_{1-4}$-alkoxy or $C_{1-4}$-alkanoyl; or (b) a quinoline residue of the formula

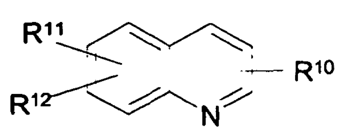

where one of $R^{10}$, $R^{11}$ and $R^{12}$ is an -NH group via which the quinoline residue is linked to the Arg residue,

another can be OH or amino, and the other can be hydrogen, OH or amino, and the salts thereof.

2. Compounds according to claim 1, wherein $R^5$ is methyl, isopropyl, phenyl, tert.-butylphenyl, 4-methylphenyl, 2,4,6-trimethylphenyl, 2,4,6-triisopropylphenyl, 4-methoxy-2,3,6-trimethylphenyl, anthraquinonyl, 1- or 2-naphthyl, quinolyl or isoquinolyl or a radical derived from camphor, or $R^6$, if representing heteroarylalkyl, is imidazolylmethyl or indolylmethyl and/or in which $R^8$ is OH in the o or p position and $R^9$ is halogen in the m position or $R^8$ is OH in the o, m or p position and $R^9$ is nitro or $C_{1-4}$-alkanoyl in the m or p position.

3. Compounds according to claim 1 or 2, wherein the N-terminal amino acid has a protective group selected from tert.-butoxycarbonyl ("Boc"), p-toluenesulfonyl ("Tos"), tert.-butylphenylsulfonyl ("t-Bups"), methylsulfonyl ("Mes"), naphthylsulfonyl ("Naps"), benzoyl ("Bzo"), benzyloxycarbonyl ("Z"), isopropylsulfonyl and camphorsulfonyl.

4. Compounds according to one of the claims 1 to 3, wherein, apart from the C-terminal amino acid L-arginine, the compound comprises 2-aminobutyric acid, alanine, 3-cyclohexylalanine, 2-cyclohexylglycine, phenylalanine, pipecolic acid, proline or valine, these amino acids possibly being in the L, D or DL form, or glycine.

5. Compounds according to claim 4, wherein, apart from the C-terminal amino acid L-arginine, the compound comprises L-alanine ("Ala"), L-2-aminobutyric acid ("Abu"), D-3-cyclohexylalanine ("D-Cha"), D-2-cyclohexylglycine ("D-Chg"), glycine ("Gly") or L-proline ("Pro").

6. Compounds according to one of the claims 1 to 5, in which $R^4$ is derived from 2-amino-4-nitrophenol, 4-amino-2-nitrophenol, 4-amino-3-nitrophenol, 2-amino-5-nitrophenol, 2,4-diaminophenol, 4-amino-m-cresol, 2-amino-4-chlorophenol, 4-amino-2-chlorophenol, 4-amino-3-chlorophenol, 4-fluoro-2-aminophenol, 2-fluoro-4-aminophenol, 5-fluoro-2-aminophenol, 5-amino-8-hydroxyquinoline or 2-amino-8-hydroxyquinoline.

7. H-(D)-Chg-Gly-Arg-3-chloro-4-hydroxyanilide;

Boc-(D)-Chg-Gly-Arg-3-chloro-4-hydroxyanilide;
Tos-(D)-Chg-Gly-Arg-3-chloro-4-hydroxyanilide;
t-Bups-(D)-Chg-Gly-Arg-3-chloro-4-hydroxyanilide;
Mes-(D)-Chg-Gly-Arg-3-chloro-4-hydroxyanilide;
Naps-2-(D)-Chg-Gly-Arg-3-chloro-4-hydroxyanilide;
Z-(D)-Chg-Gly-Arg-3-chloro-4-hydroxyanilide;
H-(D)-Cha-Gly-Arg-3-chloro-4-hydroxyanilide;
Boc-(D)-Cha-Gly-Arg-3-chloro-4-hydroxyanilide;
H-Gly-Pro-Arg-3-chloro-4-hydroxyanilide;
Boc-Gly-Pro-Arg-3-chloro-4-hydroxyanilide;
Tos-Gly-Pro-Arg-3-chloro-4-hydroxyanilide;
t-Bups-Gly-Pro-Arg-3-chloro-4-hydroxyanilide;
Mes-Gly-Pro-Arg-3-chloro-4-hydroxyanilide;
Isopropylsulfonyl-Gly-Pro-Arg-3-chloro-4-hydroxyanilide;
Naps-2-Gly-Pro-Arg-3-chloro-4-hydroxyanilide;
(-)-Camphorsulfonyl-Gly-Pro-Arg-3-chloro-4-hydroxyanilide;
H-(D)-Cha-Pro-Arg-3-chloro-4-hydroxyanilide;
Boc-(D)-Cha-Pro-Arg-3-chloro-4-hydroxyanilide;
H-(D)-Cha-Ala-Arg-3-chloro-4-hydroxyanilide;
Boc-(D)-Cha-Ala-Arg-3-chloro-4-hydroxyanilide;
Boc-(D)-Cha-Abu-Arg-3-chloro-4-hydroxyanilide;
Z-Gly-Pro-Arg-2-chloro-4-hydroxyanilide;
Z-Gly-Pro-Arg-5-chloro-2-hydroxyanilide;
Z-Gly-Pro-Arg-8-hydroxyquinolin-5-ylamide;
Boc-(D)-Chg-Gly-Pro-Arg-3-chloro-4-hydroxyanilide;
H-(D)-Chg-Gly-Pro-Arg-3-chloro-4-hydroxyanilide; and
t-Bups-(D)-Chg-Gly-Pro-Arg-3-chloro-4-hydroxyanilide.

8. Salts of compounds according to one of the claims 1 to 7 with HCl, HBr, $H_2SO_4$ or $H_3PO_4$ or with formic, acetic, propionic, phthalic, citric, oxalic, tartaric, benzoic, lactic, trichloroacetic or trifluoroacetic acid.

**9.** Salts according to claim 8 with HCl, acetic acid or trifluoroacetic acid.

**10.** A process for preparing compounds and salts thereof according to one of the claims 1 to 9, comprising: linking - according to usual methods of peptide chemistry - an amine of the formula H-$R^4$, wherein $R^4$ has the denotation given in claim 1, to the carboxyl group of arginine, whose amino group is protected or already has an appropriately protected residue of the peptide part of the desired product or a part thereof, and whose arginine group is protected; optionally assembling the peptide part of the desired product completely; optionally cleaving off the remaining protective group(s); optionally acylating a free amino group; and/or optionally converting a resulting oligopeptide derivative of the formula I defined in claim 1 into an acid addition salt; and/or converting a resulting acid addition salt of such an oligopeptide derivative into a free oligopeptide derivative or into another salt.

**11.** A method for the quantitative determination of a protease or antiprotease, comprising contacting the enzyme in an aqueous or organic medium with an oligopeptide derivative or a salt thereof according to one of the claims 1 to 9; and determining by amperometry the electroactive amine split by the enzyme to be determined of the formula H-$R^4$, wherein $R^4$ has the denotation given in claim 1; the amine can be electrochemically oxidized or reduced.

**12.** A method according to claim 11, comprising contacting a protease or antiprotease of the blood coagulation system, of the fibrinolytic system or of the complement system, in particular thrombin, as the enzyme with the oligopeptide derivative or a salt thereof.

**13.** A method according to claim 11 or 12, wherein the amperometry is carried out using an apparatus having a potentiostat and a measuring cell with two or three electrodes, a measuring electrode made of steel or a noble metal, such as platinum or gold, a reference electrode and/or an auxiliary electrode, to which the oligopeptide derivative or a salt thereof according to one of the claims 1 to 9 and optionally water-soluble additives, such as $Ca^{++}$ salts, phospholipids or thromboplastin reagents, are applied.

**14.** A method according to claim 13, wherein a mixture of 0-35% of L-α-phosphatidylcholine (PC) and 65-100% of L-α-phosphatidyl-L-serine (PS) is used as phospholipids.

**15.** A method according to claim 13, wherein the mixture comprises 25-35% PC and 65-75% PS.

**16.** A method according to one of the claims 13 to 15, wherein a change in the concentration of the electroactive amine in the measuring cell is determined through measuring an oxidation or reduction current.

**17.** A method according to one of the claims 11 to 16 for determining thrombin in whole blood, wherein the whole blood is applied directly, without removal of blood cells and without removal of other blood constituents, onto the electrode to which the oligopeptide derivative or a salt thereof according to one of the claims 1 to 9 and optionally water-soluble additives are adhering.

## Revendications

**1.** Dérivés oligopeptidiques de formule (I)

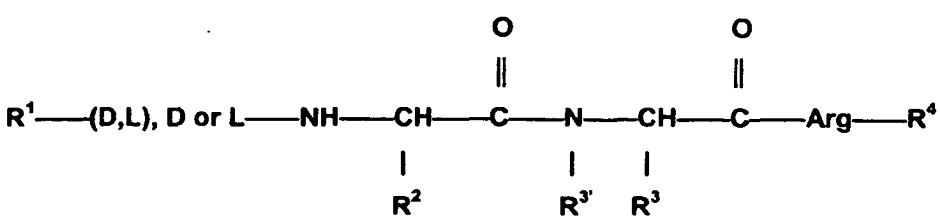

où

$R^1$ représente

(a) un atome d'hydrogène, un groupe $C_{2-8}$-alcanoyle portant éventuellement un groupe amino en position ω, un groupe phényl-$C_{2-4}$-alcanoyle dont le radical phényle est éventuellement substitué par un groupe amino en position p; ou

(b) un groupe cyclohexylcarbonyle éventuellement substitué par un radical aminométhyle en position 4, un groupe benzoyle éventuellement substitué par méthyle, amino ou halogène en position o ou p, un groupe $C_{1-8}$-alkoxycarbonyle, un groupe benzyloxycarbonyle éventuellement substitué par méthoxy, méthyle ou chlore en position p; ou

(c) un radical de formule $-SO_2-R^5$ où $R^5$ peut être un radical $C_{1-6}$-alkyle, un radical aryle ou hétéroaryle éventuellement substitué ou un radical d'un dérivé de terpène bicyclique; ou

(d) un groupe de formule $-CO-CH(R^6)-NH-R^7$, où $R^6$ représente de l'hydrogène, un radical $C_{1-6}$-alkyle, un radical 1- ou 2-hydroxyéthyle, un radical méthylmercaptoéthyle, un radical aminobutyle, un radical guanidinopropyle, un radical carboxy-$C_{1-4}$-alkyle, un radical carboxamido-$C_{1-4}$-alkyle, un radical phényl-$C_{1-4}$-alkyle dont le radical phényle est éventuellement substitué par OH, halogène, $C_{1-4}$-alkyle ou méthoxy, un radical cyclohexyle ou cyclohexylméthyle dont le cycle est éventuellement substitué par OH, halogène, $C_{1-4}$-alkyle ou méthoxy, ou un radical hétéroaryl-$C_{1-4}$-alkyle contenant de l'azote et ayant de 3 à 8 atomes de carbone dans le système hétérocyclique, le groupe $-CO-CH(R^6)-NH-R^7$pouvant être racémique ou de configuration D ou L, et $R^7$peut désigner un groupe du type (a), (b) ou (c); ou

(e) un groupe de formule

$R^7$ =

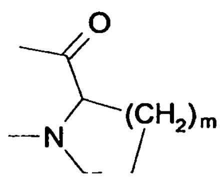

où $R^7$ possède la signification mentionnée ci-dessus, m peut être égal à 1 ou 2, et l'un des groupes méthylènes peut être substitué par OH, carboxyle, $C_{1-4}$-alkyle ou aryl-$C_{1-4}$-alkyle;

$R^2$ représente de l'hydrogène, $C_{1-6}$-alkyle, $C_{1-2}$-hydroxyalkyle, $C_1$-$_4$-alkoxy-$C_{1-6}$-alkyle, benzyloxy-$C_{1-2}$-alkyle, un radical ω-carboxy-$C_{1-3}$-alkyle, un radical ω-$C_{1-4}$-alkoxycarbonyl-$C_{1-3}$-alkyle, un radical ω-benzyloxycarbonyl-$C_{1-3}$-alkyle ou un radical cyclohexyle, cyclohexylméthyle, 4-hydroxycyclohexylméthyle, phényle, benzyle, 4-hydroxybenzyle ou imidazolyl-4-méthyle;

$R^3$ représente

(a) de l'hydrogène ou $C_{1-4}$-alkyle et $R^{3'}$ est de l'hydrogène; ou

(b) associé à $R^{3'}$ un groupe tri- ou tétraméthylène, l'un des groupes méthylènes pouvant être substitué par OH, carboxyle, $C_{1-4}$-alkyle ou aryl-$C_{1-4}$-alkyle; et

$R^4$ représente

(a) un radical aniline de formule

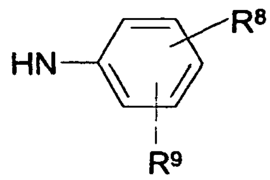

où $R^8$ peut désigner OH et $R^9$ peut être de l'hydrogène, halogène, amino, nitro, $C_{1-4}$-alkyle, $C_{1-4}$-alkoxy ou $C_{1-4}$-alkanoyle; ou

(b) un radical quinoline de formule

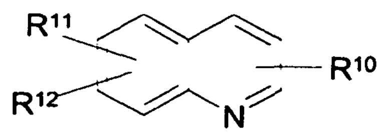

où l'un de $R^{10}$, $R^{11}$ et $R^{12}$ est un groupe -NH par lequel le radical quinoline est lié au radical Arg, un second peut être OH ou amino, et le troisième peut être l'hydrogène, OH ou amino, et leurs sels.

2. Composés selon la revendication 1, **caractérisés en ce que** $R^5$ représente méthyle, isopropyle, phényle, tert. butylphényle, 4-méthylphényle, 2,4,6-triméthylphényle, 2,4,6-triisopropylphényle, 4-méthoxy-2,3,6-triméthylphényle, anthraquinonyle, 1- ou 2-naphtyle, quinolyle ou isoquinolyle ou un radical dérivé de camphre, ou $R^6$, s'il représente hétéroarylalkyle, est de l'imidazolylméthyle ou indolylméthyle et/ou dans lesquels $R^8$ désigne OH en position o ou p et $R^9$ est de l'halogène en position m ou $R^8$ désigne OH en position o, m ou p et $R^9$ désigne nitro ou $C_{1-4}$-alcanoyle en position m ou p.

3. Composés selon la revendication 1 ou 2, **caractérisés en ce que** l'acide aminé N-terminal possède un groupe protecteur sélectionné parmi tert.-butoxycarbonyle ("Boc"), p-toluènesulfonyle ("Tos"), tert.-butylphénylsulfonyle ("t-Bups"), méthylsulfonyle ("Mes"), naphtylsulfonyle ("Naps"), benzoyle ("Bzo"), benzyloxycarbonyle ("Z"), isopropylsulfonyle et camphre-sulfonyle.

4. Composés selon l'une des revendications 1 à 3, **caractérisés en ce que** la molécule contient, outre l'acide aminé C-terminal L-arginine, de l'acide 2-aminobutyrique, de l'alanine, de la 3-cyclohexylalanine, de la 2-cyclohexylglycine, de la phénylalanine, de l'acide pipécolique, de la proline ou de la valine, ces acides aminés pouvant être configurés en L, D ou DL, ou de la glycine.

5. Composés selon la revendication 4, **caractérisés en ce que** la molécule contient, outre l'acide aminé C-terminal L-arginine, de la L-alanine ("Ala"), de l'acide L-2-aminobutyrique ("Abu"), de la D-3-cyclohexylalanine ("D-Cha"), de la D-2-cyclohexylglycine ("D-Chg"), de la glycine ("Gly") ou de la L-proline ("Pro").

6. Composés selon l'une des revendications 1 à 5, **caractérisés en ce que** $R^4$ est dérivé de 2-amino-4-nitrophénol, 4-amino-2-nitrophénol, 4-amino-3-nitrophénol, 2-amino-5-nitrophénol, 2,4-diaminophénol, 4-amino-m-crésol, 2-amino-4-chlorophénol, 4-amino-2-chlorophénol, 4-amino-3-chlorophénol, 4-fluoro-2-aminophénol, 2-fluoro-4-aminophénol, 5-fluoro-2-aminophénol, 5-amino-8-hydroxyquinoline ou 2-amino-8-hydroxyquinoline.

7. H-(D)-Chg-Gly-Arg-3-chloro-4-hydroxyanilide;

Boc-(D)-Chg-Gly-Arg-3-chloro-4-hydroxyanilide;
Tos-(D)-Chg-Gly-Arg-3-chloro-4-hydroxyanilide;
t-Bups-(D)-Chg-Gly-Arg-3-chloro-4-hydroxyanilide;
Mes-(D)-Chg-Gly-Arg-3-chloro-4-hydroxyanilide;
Naps-2-(D)-Chg-Gly-Arg-3-chloro-4-hydroxyanilide;
Z-(D)-Chg-Gly-Arg-3-chloro-4-hydroxyanilide;

H-(D)-Cha-Gly-Arg-3-chloro-4-hydroxyanilide;
Boc-(D)-Cha-Gly-Arg-3-chloro-4-hydroxyanilide;
H-Gly-Pro-Arg-3-chloro-4-hydroxyanilide;
Boc-Gly-Pro-Arg-3-chloro-4-hydroxyanilide;
Tos-Gly-Pro-Arg-3-chloro-4-hydroxyanilide;
t-Bups-Gly-Pro-Arg-3-chloro-4-hydroxyanilide;
Mes-Gly-Pro-Arg-3-chloro-4-hydroxyanilide;
Isopropylsulfonyl-Gly-Pro-Arg-3-chloro-4-hydroxyanilide;
Naps-2-Gly-Pro-Arg-3-chloro-4-hydroxyanilide;
(-)-Camphre-sulfonyl-Gly-Pro-Arg-3-chloro-4-hydroxyanilide;
H-(D)-Cha-Pro-Arg-3-chloro-4-hydroxyanilide;
Boc-(D)-Cha-Pro-Arg-3-chloro-4-hydroxyanilide;
H-(D)-Cha-Ala-Arg-3-chloro-4-hydroxyanilide;
Boc-(D)-Cha-Ala-Arg-3-chloro-4-hydroxyanilide;
Boc-(D)-Cha-Abu-Arg-3-chloro-4-hydroxyanilide;
Z-Gly-Pro-Arg-2-chloro-4-hydroxyanilide;
Z-Gly-Pro-Arg-5-chloro-2-hydroxyanilide;
Z-Gly-Pro-Arg-8-hydroxyquinolin-5-ylamide;
Boc-(D)-Chg-Gly-Pro-Arg-3-chloro-4-hydroxyanilide;
H-(D)-Chg-Gly-Pro-Arg-3-chloro-4-hydroxyanilide; et
t-Bups-(D)-Chg-Gly-Pro-Arg-3-chloro-4-hydroxyanilide.

**8.** Sels de composés selon l'une des revendications 1 à 7 avec du HCl, HBr, $H_2SO_4$ ou $H_3PO_4$ ou avec de l'acide formique, acétique, propionique, phtalique, citrique, oxalique, tartrique, benzoïque, lactique, trichloracétique ou trifluoracétique.

**9.** Sels selon la revendication 8 avec du HCl, de l'acide acétique ou de l'acide trifluoracétique.

**10.** Procédé de fabrication de composés et de leurs sels selon l'une des revendications 1 à 9, **caractérisé en ce qu'**on couple, suivant des méthodes couramment utilisées dans la chimie des peptides, une amine de formule $H-R^4$, où $R^4$ possède la signification donnée dans la revendication 1, au groupe carboxyle de l'arginine, dont le groupe amino est protégé ou porte déjà le radical protégé de manière appropriée de la partie peptidique du produit désiré ou une partie de ce produit, et dont le groupe arginine est protégé; qu'on assemble éventuellement la partie peptidique du produit désiré dans sa totalité; qu'on scinde éventuellement le(s) groupe(s) protecteur(s) restant(s); qu'on acyle éventuellement un groupe amino libre; et/ou qu'on transforme éventuellement un dérivé oligopeptidique obtenu de formule I définie dans la revendication 1 en un sel d'addition acide; et/ou qu'on transforme un sel d'addition acide obtenu d'un tel dérivé oligopeptidique en un dérivé oligopeptidique libre ou en un autre sel.

**11.** Méthode pour le dosage quantitatif d'une protéase ou antiprotéase, **caractérisée en ce qu'**on met en contact dans un milieu aqueux ou organique l'enzyme et un dérivé oligopeptidique ou un de ses sels selon l'une des revendications 1 à 9 et qu'on détermine par ampérométrie l'amine électroactive scindée par l'enzyme à déterminer de formule $H-R^4$, où $R^4$ possède la signification donnée dans la revendication 1, l'amine pouvant être oxydée ou réduite par électrochimie.

**12.** Méthode selon la revendication 11, **caractérisée en ce** quon met en contact une protéase ou antiprotéase du système de la coagulation sanguine, du système fibrinolytique ou du système complémentaire, en particulier la thrombine, en tant qu'enzyme et le dérivé oligopeptidique ou un de ses sels.

**13.** Méthode selon la revendication 11 ou 12, **caractérisée en ce que** l'ampèremètre est muni d'un potentiostat et d'un capteur avec deux ou trois électrodes, d'une électrode de mesure en acier ou en métal noble comme le platine ou l'or, d'une électrode de référence et/ou d'une électrode auxiliaire, le dérivé oligopeptidique ou l'un de ses sels selon l'une des revendications 1 à 9 et éventuellement des additifs hydrosolubles, tels que des sels de $Ca^{++}$, des phospholipides ou des réactifs de thromboplastine, étant appliqué(s) sur les électrodes.

**14.** Méthode selon la revendication 13, **caractérisée en ce qu'**on utilise comme phospholipides un mélange de 0-35% de L-$\alpha$-phosphatidyl-choline (PC) et de 65-100% de L-$\alpha$-phosphatidyl-L-sérine (PS).

**15.** Méthode selon la revendication 13, **caractérisée en ce que** le mélange contient 25-35% de PC et 65-75% de PS.

**16.** Méthode selon l'une des revendications 13 à 15, **caractérisée en ce que** la modification de la concentration en amine électroactive dans le capteur est déterminée par le courant d'oxydation ou de réduction mesuré.

**17.** Méthode selon l'une des revendications 11 à 16 pour la détermination de thrombine dans du sang entier, **caractérisée en ce que** le sang entier, sans séparation des globules sanguins et sans séparation d'autres constituants sanguins, est directement appliqué sur l'électrode à laquelle adhèrent le dérivé oligopeptidique ou l'un de ses sels selon l'une des revendications 1 à 9 et éventuellement des additifs hydrosolubles.

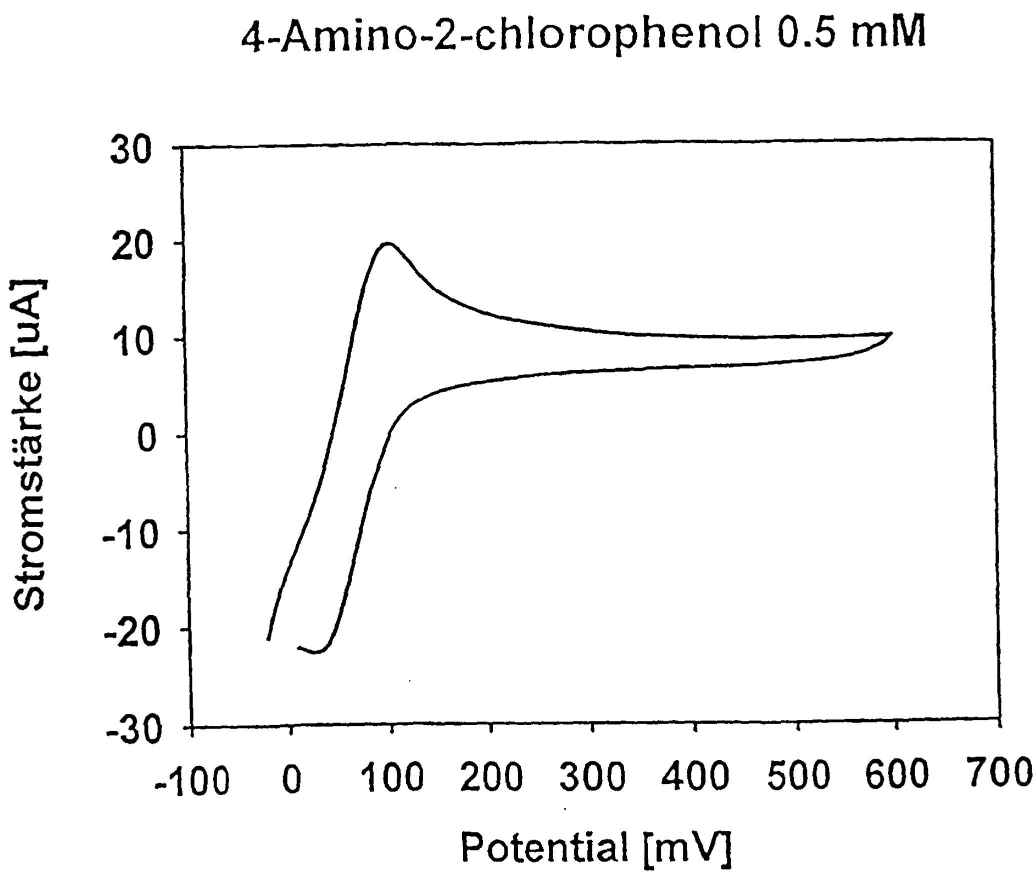
4-Amino-2-chlorophenol 0.5 mM
30
20
10
0
-10
-20
-30
Stromstärke [uA]
-100
0
100
200
300
400
500
600
700
Potential [mV]

Fig. 1

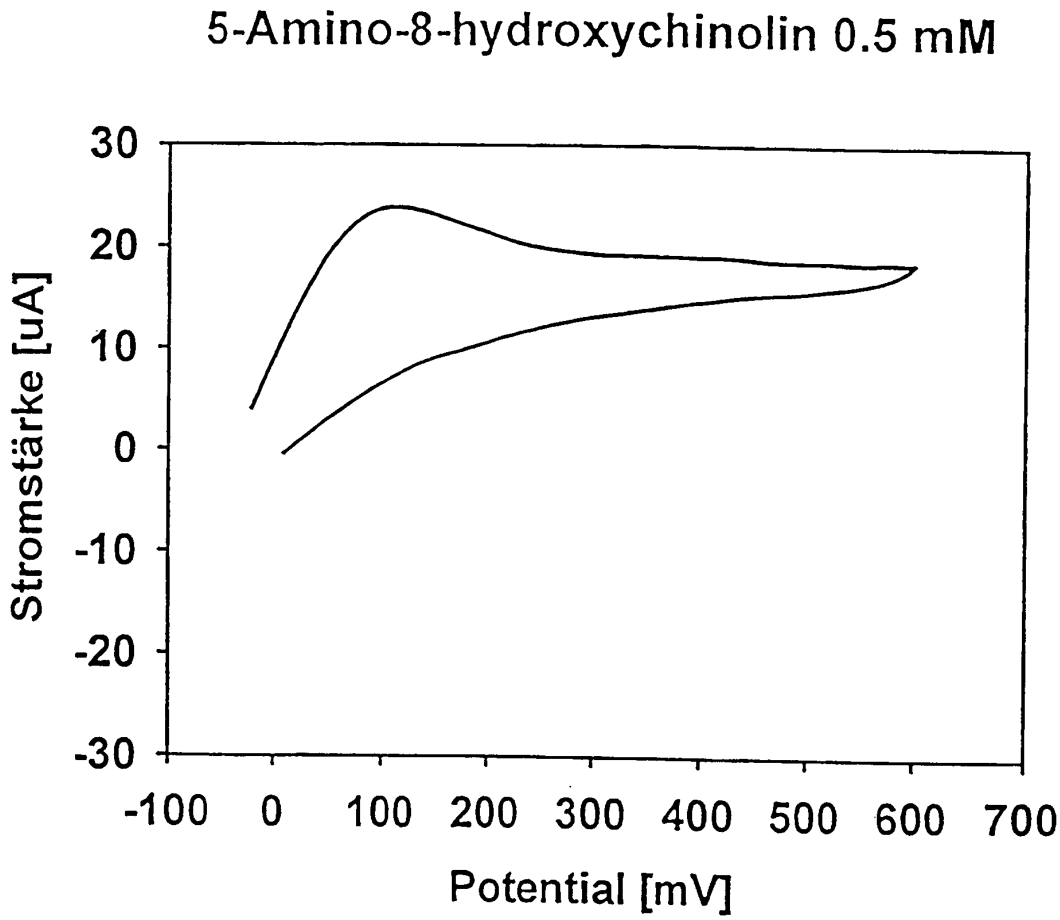
5-Amino-8-hydroxychinolin 0.5 mM
30
20
10
0
-10
-20
-30
Stromstärke [uA]
-100
0
100
200
300
400
500
600
700
Potential [mV]

Fig. 2

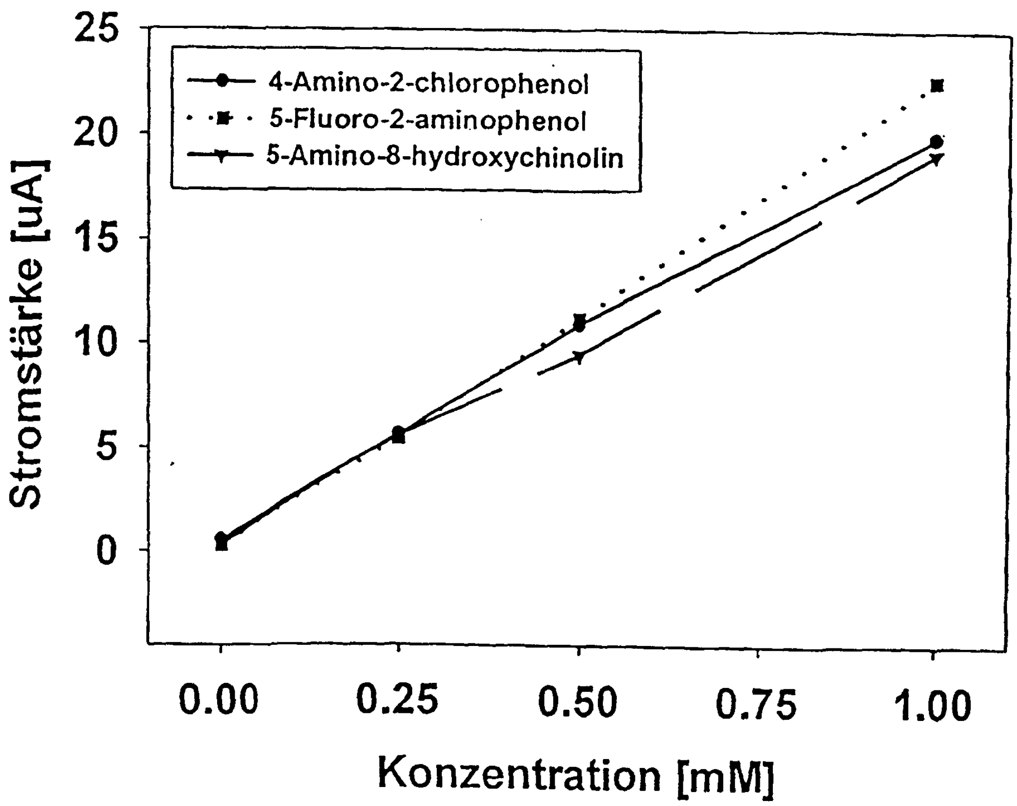
4-Amino-2-chlorophenol
5-Fluoro-2-aminophenol
5-Amino-8-hydroxychinolin
Stromstärke [uA]
25
20
15
10
5
0
0.00
0.25
0.50
0.75
1.00
Konzentration [mM]

Fig. 3

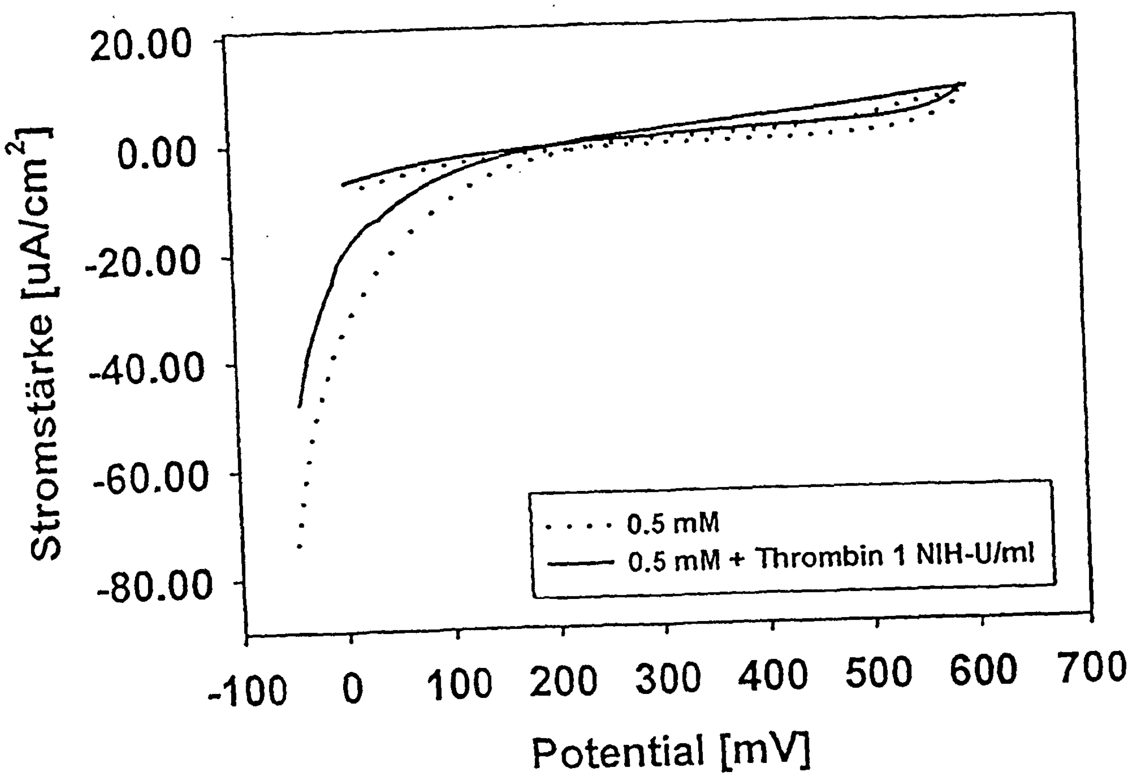
20.00
0.00
-20.00
-40.00
-60.00
-80.00
Stromstärke [uA/cm2]
-100
0
100
200
300
400
500
600
700
Potential [mV]
0.5 mM
0.5 mM + Thrombin 1 NIH-U/ml

Fig. 4

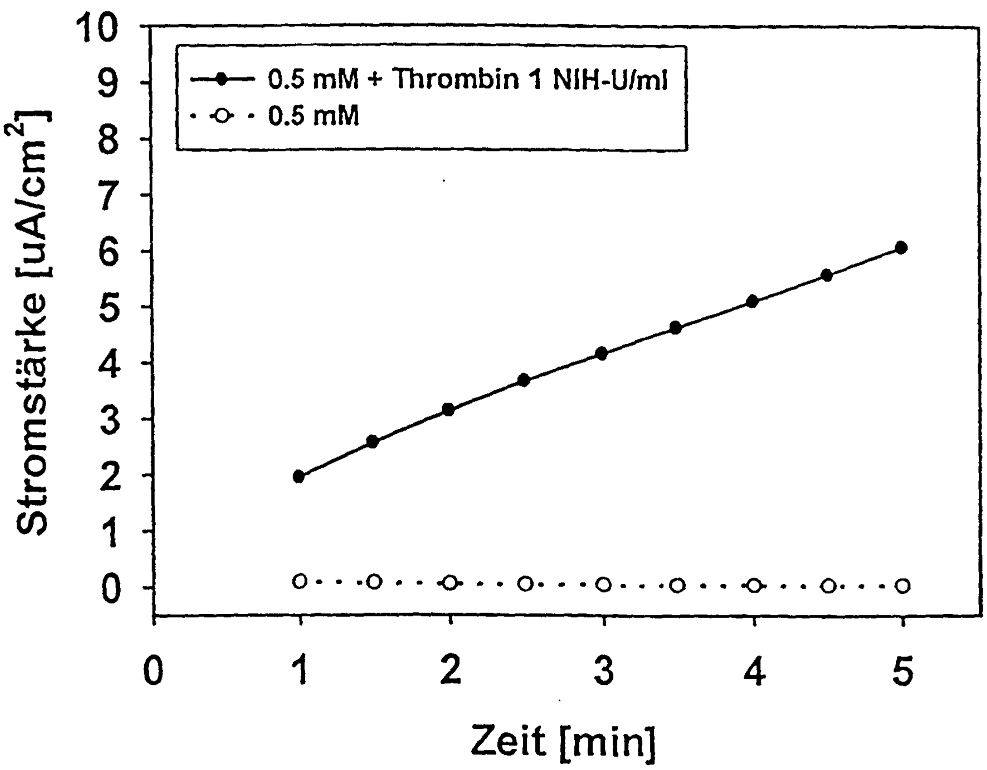
10
9
8
7
6
5
4
3
2
1
0
Stromstärke [uA/cm2]
0.5 mM + Thrombin 1 NIH-U/ml
0.5 mM
0
1
2
3
4
5
Zeit [min]

Fig. 5

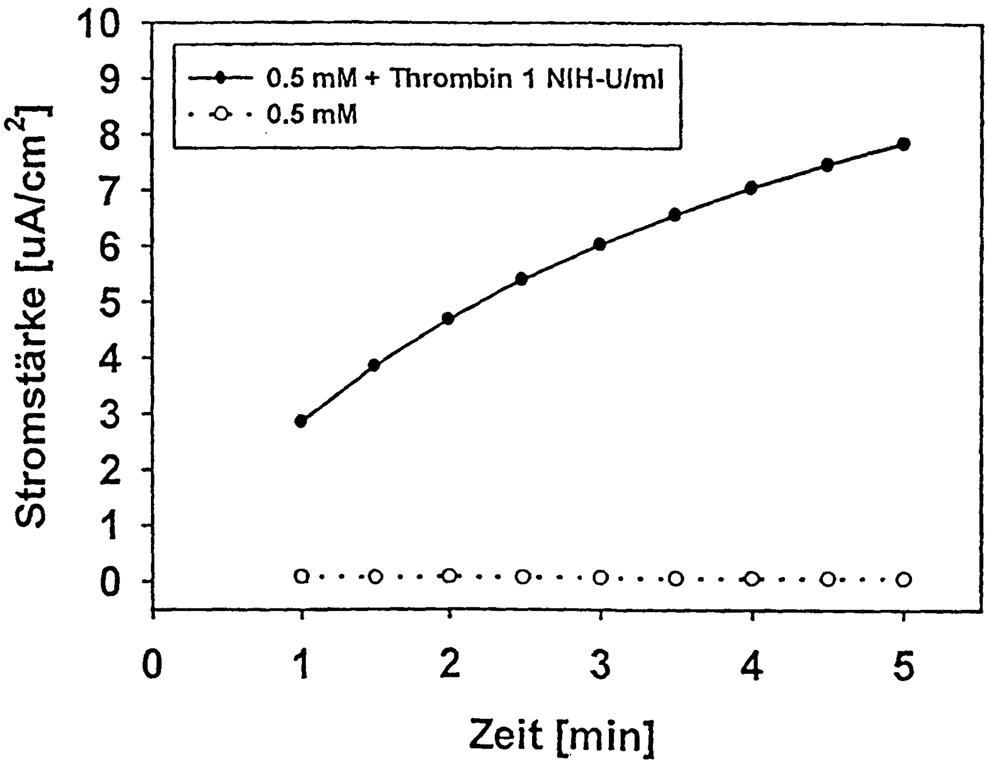
10
9
8
7
6
5
4
3
2
1
0
Stromstärke [uA/cm2]
0.5 mM + Thrombin 1 NIH-U/ml
0.5 mM
0
1
2
3
4
5
Zeit [min]

Fig. 6

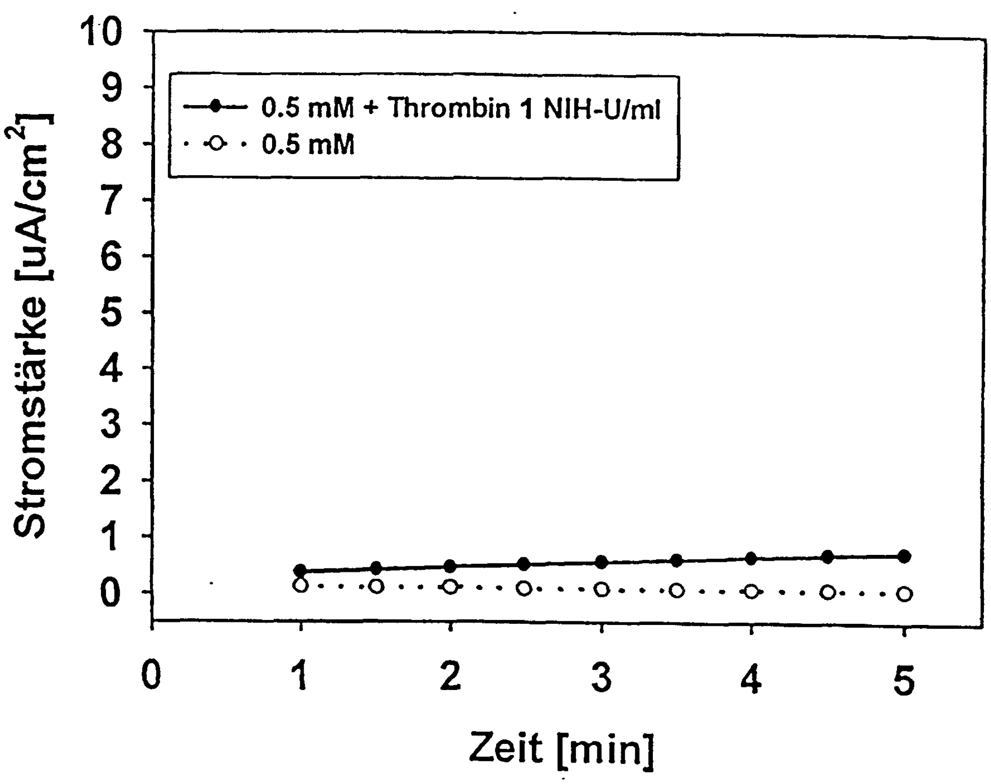
10
9
8
7
6
5
4
3
2
1
0
Stromstärke [uA/cm2]
0.5 mM + Thrombin 1 NIH-U/ml
0.5 mM
0
1
2
3
4
5
Zeit [min]

Fig. 7

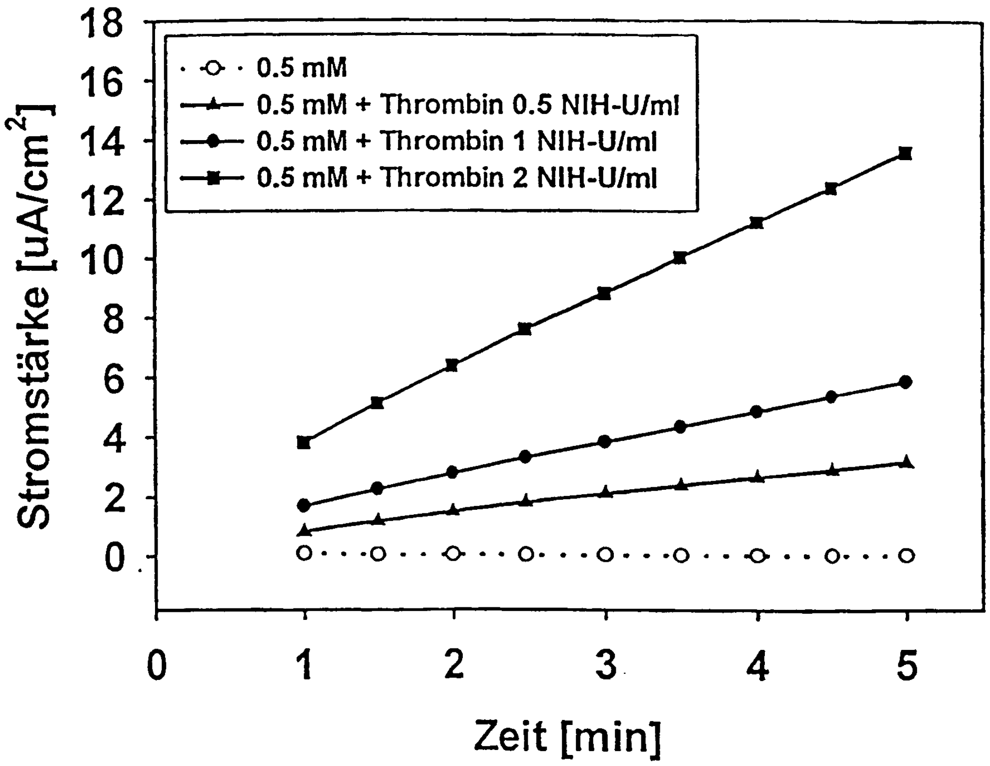
18
16
14
12
10
8
6
4
2
0
Stromstärke [uA/cm2]
0.5 mM
0.5 mM + Thrombin 0.5 NIH-U/ml
0.5 mM + Thrombin 1 NIH-U/ml
0.5 mM + Thrombin 2 NIH-U/ml
0
1
2
3
4
5
Zeit [min]

Fig. 8

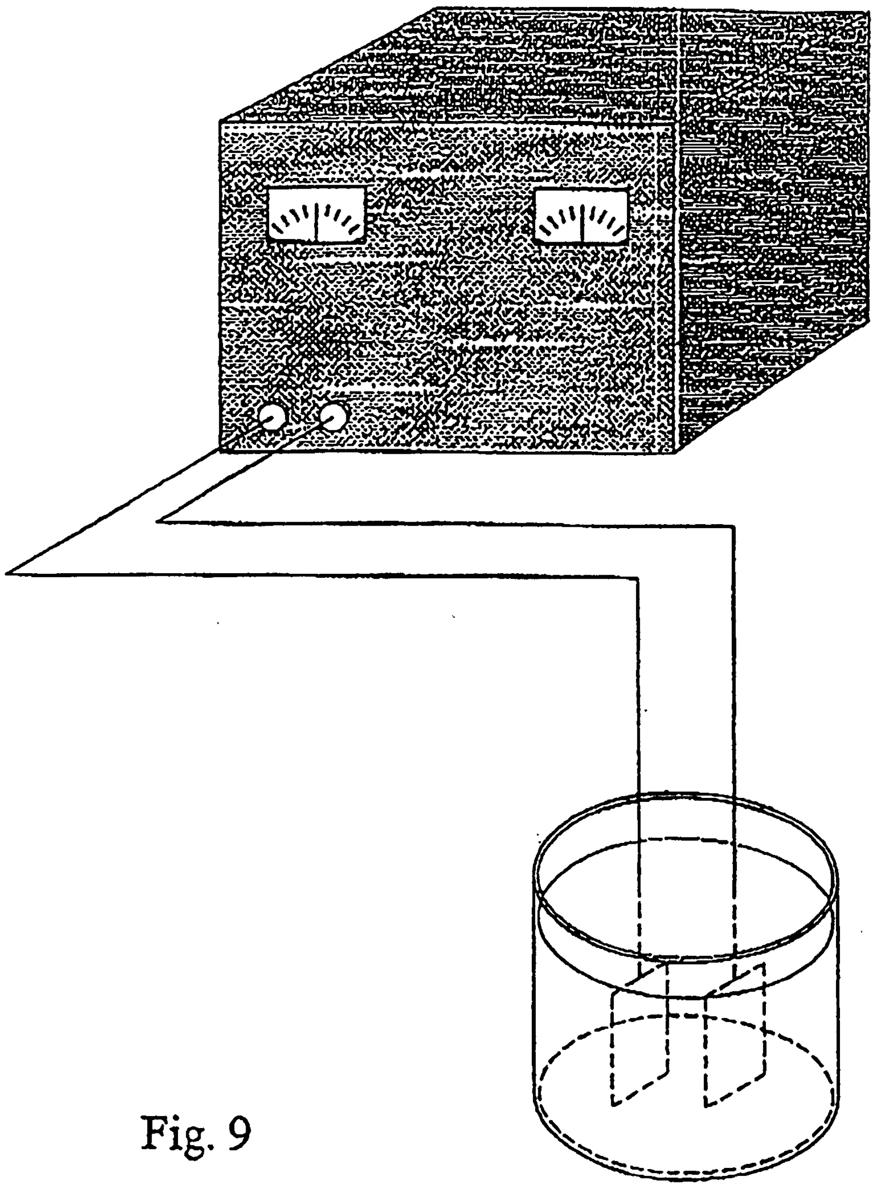

Fig. 9

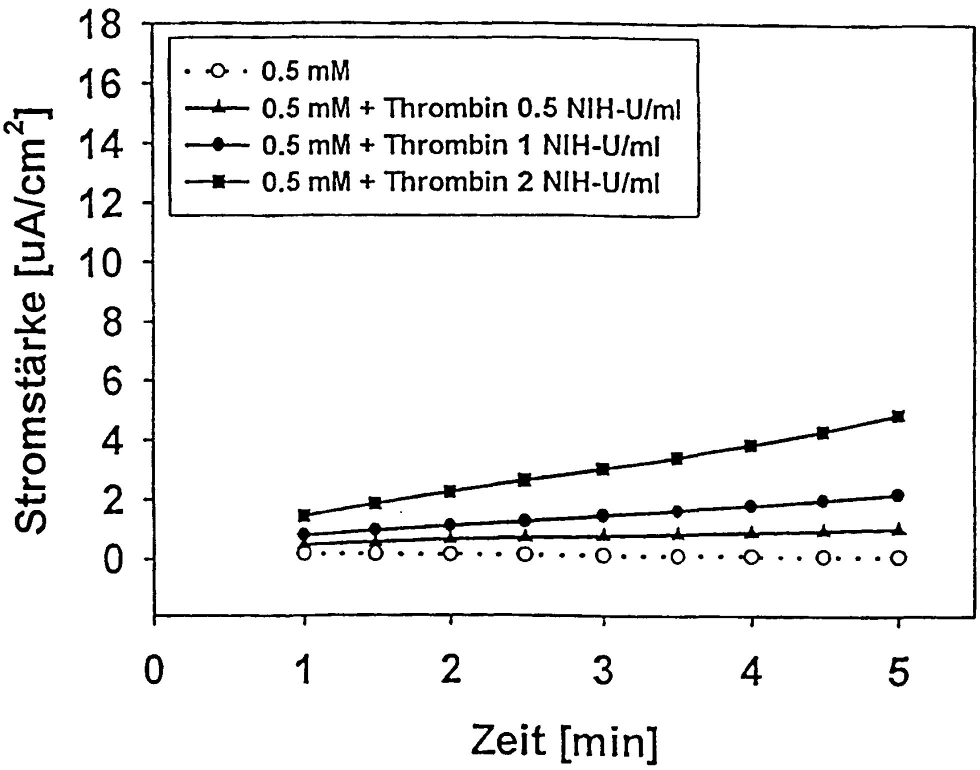
0.5 mM
0.5 mM + Thrombin 0.5 NIH-U/ml
0.5 mM + Thrombin 1 NIH-U/ml
0.5 mM + Thrombin 2 NIH-U/ml
Stromstärke [uA/cm$^2$]
18
16
14
12
10
8
6
4
2
0
0
1
2
3
4
5
Zeit [min]

Fig. 10

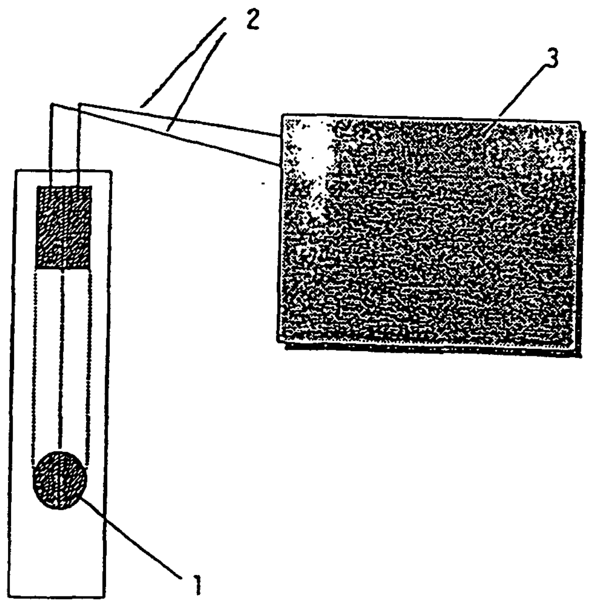


Fig. 11

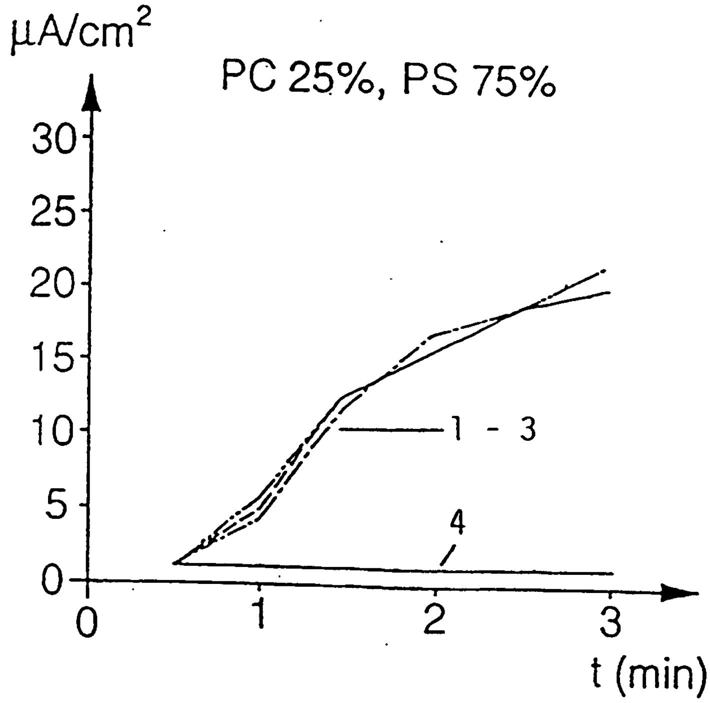


Fig. 12